# EUROPEAN PATENT APPLICATION

(11) **EP 4 737 477 A2**
(43) Date of publication of application: **06.05.2026**
(21) Application number: 26151778.3
(22) Date of filing: 14.06.2022
(51) Int. Cl.: C07K 14/575

(54) **HEPCIDIN MIMETICS FOR TREATMENT OF HEREDITARY HEMOCHROMATOSIS**

(30) Priority: 14.06.2021 US 202163210453 P; 04.10.2021 US 202163252001 P; 07.06.2022 US 202263349841 P
(62) Divisional of application: 22825644.2
(71) Applicant: Protagonist Therapeutics, Inc., Newark, CA 94560-1160 (US)
(72) Inventor: GUPTA, Suneel Kumar, Sunnyvale, 94087 (US); LIU, David Y., Newark, 94560 (US); MODI, Nishit Bachulal, Sunnyvale, 94087 (US); VALONE, Frank Horace, Newark, 94560 (US)
(74) Representative: Carpmaels & Ransford LLP

(57) **Abstract**

The disclosure provides methods for the treatment and/or prevention of iron overload diseases such as hereditary hemochromatosis.

## Description

### RELATED APPLICATIONS

This application claims priority to U.S. Provisional Patent Application Serial No. 63/210,453, filed June 14, 2021, U.S. Provisional Patent Application Serial No. 63/252,001, filed October 4, 2021, and U.S. Provisional Patent Application Serial No. 63/349,841, filed June 7, 2022, which is incorporated herein by reference in their entireties.

### SEQUENCE LISTING

This application is being filed electronically via EFS-Web and includes an electronically submitted sequence listing in .txt format. The .txt file contains a sequence listing entitled PRTH_070_02WO _ST25.txt created on June 13, 2022 and having a size of 24 kilobytes. The sequence listing contained in this .txt file is part of the specification and is incorporated herein by reference in its entirety.

### FIELD OF THE INVENTION

The present disclosure relates, inter alia, to methods for the treatment and/or prevention of iron overload diseases, such as hereditary hemochromatosis.

### BACKGROUND

Iron plays an important role in many cellular and organismal activities, from cell division to oxygen transport (see, e.g., Casu, C. et al., Blood 2018: 131(16): 1790-1794). However, excess iron promotes the formation of toxic reactive oxygen species (ROS), which can damage DNA, proteins, and lipid membranes, and lead to organ dysfunction or failure, and humans and other vertebrates have evolved regulatory systems to optimize the absorption and organ distribution of iron. Unfortunately, however, several genetic or acquired disorders of iron homeostasis dysregulate iron absorption or distribution, causing organ damage and creating conditions for overwhelming infections or inflammation, with associated morbidity and mortality.

Hepcidin is a 25 amino acid peptide hormone produced primarily in the liver in proportion to plasma iron concentration and iron stores. Hepcidin is a key regulator of iron homeostasis, since it binds and degrades the only known iron exporter, ferroportin-1, which is expressed on the surfaces of cells involved in iron absorption, recycling, and storage.

Given the central role of iron homeostasis in a variety of diseases and disorders, agents capable of modulating iron levels, e.g., in erythrocytes and organs, are being developed as therapeutic agents. However, there remains a need for methods for using iron modulating agents to restore iron homeostasis, e.g., for the treatment of hereditary hemochromatosis. The present invention addresses this need.

### SUMMARY OF THE INVENTION

The present disclosure provides methods, clinically effective dosages and dosing regimens of hepcidin mimetics that restore iron homeostasis in human, e.g., humans with hereditary hemochromatosis. In certain embodiments, the methods modulate pharmacodynamic markers associated with efficacy in the treatment of disease and disorders associated with dysregulation of iron homeostasis, such iron overload diseases and disorders, including, e.g., hereditary hemochromatosis (HH). In certain embodiments, dosages, dosage regimens, and methods disclosed herein are used to treat hereditary hemochromatosis, hereditary hemochromatosis arthropathy, or joint pain associated with hereditary hemochromatosis arthropathy.

In one aspect, the disclosure provides a method for treating an iron overload disease, e.g., HH, in a human subject, comprising providing to the subject an effective amount of a hepcidin mimetic, such as Compound 25. In certain embodiments, the subject has been diagnosed with hereditary hemochromatosis. In certain embodiments, the subject is receiving or requires phlebotomy treatment prior to the treatment disclosed herein. In particular embodiments, the subject is administered a first effective amount for a first time period, and a second effective amount for a second time period. In certain embodiments, the subject is administered a third or more effective amounts for a third or more time periods. In certain embodiments, the frequency of administration during the first and second time periods are the same or different. In certain embodiments, the frequency of administration during the third or more time periods are each the same or different from that of the first and second time period and each other. In particular embodiments, each time period independently comprises about one week, about two weeks, about four weeks, about one month, about two months, about four months, about six months, or about one year. In certain embodiments, the dosage and or frequency of administration is altered following testing of the subject's serum iron and/or TSAT saturation levels after a time period of treatment, so as to achieve parameters disclosed herein.

In a related aspect, the disclosure provides a method for treating hereditary hemochromatosis arthropathy or joint pain associated with hereditary hemochromatosis arthropathy in a human subject, comprising administering to the subject an effective amount of a hepcidin mimetic. In particular embodiments, the effective amount comprises a dose in the range of about 5 mg to about 40 mg, and optionally the subject is administered different doses during different time periods over a course of treatment. In some embodiments, the hepcidin mimetic is compound 25. In certain embodiments, an effective dose is a dose that results in the treated patients TSAT % to be reduced to ≤45% or ≤40%.

In certain embodiments of the methods disclosed herein, the effective amount of the hepcidin mimetic is about 10 mg to about 40 mg for at least some time period during the course of treatment. In particular embodiments, the effective amount of the hepcidin mimetic is administered to the subject about once a week or about twice a week for at least some time period during the course of treatment. In some embodiments, the subject is administered about 10 mg to about 15 mg, about 15 mg to about 20 mg, or about 10 mg to about 20 mg of the hepcidin mimetic about twice a week for at least some time period during the course of treatment, and in another embodiments, the subject is administered about 20 mg to about 30 mg, about 20 mg to about 40 mg, or about 30 mg to about 40 mg of the hepcidin mimetic about once a week for at least some time period during the course of treatment. In certain embodiments, the subject is administered about 5 mg, about 6 mg, about 7 mg, about 8 mg, about 9 mg, about 10 mg, about 11 mg, about 12 mg, about 13 mg, about 14 mg, about 15 mg, about 16 mg, about 17 mg, about 18 mg, about 19 mg, about 20 mg, about 21 mg, about 22 mg, about 23 mg, about 24 mg, or about 25 mg of the hepcidin mimetic about twice a week for at least some time period during the course of treatment. In certain embodiments, the subject is administered about 15 mg, about 16 mg, about 17 mg, about 18 mg, about 19 mg, about 20 mg, about 21 mg, about 22 mg, about 23 mg, about 24 mg, about 25 mg, about 26 mg, about 27 mg, about 28 mg, about 29 mg, about 30 mg, about 31 mg, about 32 mg, about 33 mg, about 34 mg, about 35 mg, about 36 mg, about 37 mg, about 38 mg, about 39 mg, about 40 mg, about 41 mg, about 42 mg, about 43 mg, about 44 mg, or about 45 mg of the hepcidin mimetic about twice a week for at least some time period during the course of treatment. In particular embodiments, the hepcidin mimetic is administered subcutaneously.

In some embodiments of any of the methods disclosed herein, the method results in the subject's TSAT level being decreased to less than about 50% TSAT (%) or less than about 45% TSAT (%) or less than about 40% TSAT (%), or less than 35% TSAT or less than 30% TSAT. In some embodiments, the method results in the subject's serum iron level being decreased to less than about 150 ug/dL. In some embodiments, the method results in the subject's liver iron concentration being substantially maintained, e.g., not significantly changed. In some embodiments, the method results in the subject's serum ferritin and serum transferrin levels or concentrations being substantially maintained, e.g., not significantly changed. In some embodiments, the method results in the subject having improved emotional and/or physical outcomes. In certain embodiments, the subject received phlebotomies for at least six months prior to treatment, optionally with a phlebotomy frequency of 0.25 - 1 phlebotomy per month, and in certain embodiments, during the treatment, the subject required substantially fewer or no phlebotomies, optionally with a phlebotomy frequency of less than 0.1, less than 0.05, or no phlebotomies per month. In certain embodiments of the disclosed methods, the subject requires reduced or no phlebotomy treatment during the course of a method of treatment disclosed herein. For example, a subject undergoing a course of treatment disclosed herein may require no phlebotomies per month, or one or less phlebotomies per month over the course of treatment disclosed herein. In particular embodiments, the subject undergoing a course of treatment disclosed herein may need fewer phlebotomies than they were receiving prior to treatment with a hepcidin mimetic according to the disclosure. In certain embodiments, the treatment according to the present disclosure results in an at least 25%, at least 50%, or at least 75% reduction in the number of phlebotomies per month. In particular embodiments, a course of a treatment disclosed herein may be for at least 12 weeks, at least 24 weeks, at least six months, at least 1 year, at least two years, at least 5 years, or longer.

In some embodiments of any of the methods disclosed herein, the hepcidin mimetic comprises a peptide of any one of Formulas I-VIII as disclosed herein. In certain embodiments, the peptide comprises or consists of one of the following sequences or structures:
Isovaleric acid-DTHFPICIFGPRSKGWVC-NH₂ (Compound 1: SEQ ID NO: 1):
Isovaleric acid-DTHFPCIIFGPRSKGWVCK-NH₂ (Compound 2: SEQ ID NO: 2);
Isovaleric acid-DTHFPCIIFEPRSKGWVCK-NH₂ (Compound 3: SEQ ID NO: 3);
Isovaleric acid-DTHFPCIIFGPRSKGWACK-NH₂ (Compound 4: SEQ ID NO: 4);
Isovaleric acid-DTHFPCIIFGPRSKGWVCKK-NH₂ (Compound 5: SEQ ID NO: 5);
Isovaleric acid-DTHFPCIIFVCHRPKGCYRRVCR-NH₂ (Compound 6; SEQ ID NO: 6);
Isovaleric acid-DTHFPCI(K(PEG8))FGPRSKGWVCK-NH₂ (Compound 7; SEQ ID NO: 7);
Isovaleric acid-DTHFPCIKF(K(PEG8))PRSKGWVCK-NH₂ (Compound 8; SEQ ID NO: 8);
Isovaleric acid-DTHFPICIFGPRS(K(PEG8))GWVC-NH₂ (Compound 9; SEQ ID NO: 9);
Isovaleric acid-DTHFPICIFGPRS(K(PEG4))GWVC-NH₂ (Compound 10; SEQ ID NO: 10);
Isovaleric acid-DTHFPCIIFGPRSRGWVC(K(PEG8))-NH₂ (Compound 11; SEQ ID NO: 11);
Isovaleric acid-DTHFPCIIFGPRSRGWVC(K(PEG4))-NH₂(Compound 12; SEQ ID NO: 12);
Isovaleric acid-DTHFPCIIFGPRSRGWVC(K(PEG2))-NH₂ (Compound 13; SEQ ID NO: 13);
Isovaleric acid-DTHFPCI(K(Palm))FGPRSKGWVCK-NH₂ (Compound 14; SEQ ID NO: 14);
Isovaleric acid-DTHFPCIKF)K(Palm))PRSKGWVCK-NH₂(Compound 15; SEQ ID NO: 15);
Isovaleric acid-DTHFPCIKFGP(K(Palm))SKGWVCK-NH₂(Compound 16; SEQ ID NO: 16);
Isovaleric acid-DTHFPCIKFGPRS(K(Palm))GWVCK-NH₂ (Compound 17; SEQ ID NO: 17);
Isovaleric acid-DTHFPCIKFGPRSKGWVC(K(Palm))NH₂ (Compound 18; SEQ ID NO: 18);
Isovaleric acid-DTHFPCI(K(PEG3-Palm))FGPRSKGWVCK-NH₂ (Compound 19; SEQ ID NO: 19);
Isovaleric acid-DTHFPCIKF(K(PEG3-Palm))PRSKGWVCK-NH₂ (Compound 20; SEQ ID NO: 20);
Isovaleric acid-DTHFPCIKFGP(K(PEG3-Palm))SKGWVCK-NH₂ (Compound 21; SEQ ID NO: 21);
Isovaleric acid-DTHFPCIKFGPRS(K(PEG3-Palm))GWVCK-NH₂ (Compound 22; SEQ ID NO: 22):
Isovaleric acid-DTHFPCIKFGPRSKGWVC(K(PEG3-Palm))-NH₂ (Compound 23; SEQ ID NO: 23);
Isovaleric acid-DTHFPCIKFGPRSKGWVC(K(PEG8))-NH₂ (Compound 24: SEQ ID NO: 24);
Isovaleric acid-DTHFPCI(K(isoGlu-Palm))FEPRSKGCK-NH₂ (Compound 25: SEQ ID NO: 25);
Isovaleric acid-DTHFPCIKF-K(isoGlu-Palm)-PRSKGCK-NH₂ (Compound 26: SEQ ID NO: 26);
Isovaleric acid-DTHFPCIKFEP(K(isoGlu-Palm))SKGCK-NH₂ (Compound 27: SEQ ID NO: 27);
Isovaleric acid-DTHFPCIKFEP(K(isoGlu-Palm))SKGWECK-NH₂ (Compound 28; SEQ ID NO: 28);
Isovaleric acid-DTHFPCIKFEPRS(K(isoGlu-Palm))GCK-NH₂ (Compound 29; SEQ ID NO: 29);
Isovaleric acid-DTHFPCIKFEPRSK(K(isoGlu-Palm))CK-NH₂ (Compound 30; SEQ ID NO: 30);
Isovaleric acid-DTHFPCIKFEPRSKGCK(K(isoGlu-Palm))-NH₂ (Compound 31; SEQ ID NO: 31);
Isovaleric acid-DTHFPCI-K(Dapa-Palm)-FEPRSKGCK-NH₂ (Compound 32; SEQ ID NO: 32);
Isovaleric acid-DTHFPCIK(F(Dapa-Palm))PRSKGCK-NH₂ (Compound 33; SEQ ID NO: 33); Isovaleric acid-DTHFPCIKFEP(K(Dapa-Palm))SKGCK-NH₂ (Compound 34; SEQ ID NO: 34);
Isovaleric acid-DTHFPCIKFEPRS(K(Dapa-Palm))GCK-NH₂ (Compound 35; SEQ ID NO: 35);
Isovaleric acid-DTHFPCIKFEPRSK(K(Dapa-Palm))CK-NH₂ (Compound 36; SEQ ID NO: 36);
Isovaleric acid-DTHFPCIKFEPRSKGC(K(Dapa-Palm))K-NH₂ (Compound 37; SEQ ID NO: 37);
Isovaleric acid-DTHFPCIKFEPRSKGC(K(Dapa-Palm))-NH₂ (Compound 38; SEQ ID NO: 38):
Isovaleric acid-DTHFPCIKF(K(PEG11-Palm))PRSK[Sar]CK-NH₂ (Compound 39; SEQ ID NO: 39):
Isolvaleric acid-DTHFPCIKF-NH₂ (Compound 40; SEQ ID NO: 40);
Hy-DTHFPCIKF-NH₂ (Compound 41; SEQ ID NO: 41);
Isolvaleric acid-DTHFPCIIF-NH₂ (Compound 42; SEQ ID NO: 42):
Hy-DTHFPCIIKF-NH₂ (Compound 43; SEQ ID NO: 43):
Isovaleric acid-DTKFPCIIF-NH₂ (Compound 44; SEQ ID NO: 44): or
Hy-DTKFPCIIF-NH₂ (Compound 45; SEQ ID NO: 45).

In particular embodiments of any of the methods disclosed herein, the reduction in the TSAT% level and/or serum iron level is the maximum reduction following treatment with the agent, while in other embodiments, the reduction in the TSAT% level and/or serum iron level is the reduction observed at trough level of the agent following administration to a subject. In some embodiments, TSAT% is reduced to less than or equal to about 45%, less than or equal to about 40%, less than or equal to about 30%, less than or equal to about 20%, less than or equal to about 15%, less than or equal to about 10%, between about 10% and about 40%, between about 5% and about 20%, or between about 10% and about 20% at the time tested. In some embodiments, the serum iron is reduced to less than or equal to about 150 microgm/dL, less than or equal to about 100 microgm/dL, less than or equal to about 75 microgm/dL less than or equal to about 600 microgm/dL at the time tested. In some embodiments, TSAT% level and/or serum iron level is reduced to a level less than 90%, less than 80%, less than 70%, less than 60%, or less than 50% of the level observed in normal, healthy volunteers.

In various embodiments of any of the methods disclosed herein, the agent, e.g., hepcidin mimetic peptide, such as Compound 25, is provided to the subject as a salt form and/or in a pharmaceutical composition, and in certain embodiments, it is provided parenterally, e.g., subcutaneously.

### BRIEF DESCRIPTION OF THE DRAWINGS

FIG. 1 provides graphs showing serum iron levels, serum ferritin levels, and total iron in various tissues following treatment of HH mice with 5 mg/kg of Compound 25.
FIGs. 2A and 2B are graphs showing non-heme iron levels in liver of HH mice following treatment with Compound 25 after a low iron diet (FIG. 2A) or a normal iron diet (FIG. 2B).
FIG. 3 outlines a phase 2 study design for treatment of hereditary hemochromatosis (HH) with a hepcidin mimetic.
FIGs. 4A and 4B show the effect of treatment with Compound 25 on phlebotomy frequency. FIG. 4A is a chart showing phlebotomy treatment of HH patients before and after treatment with a hepcidin mimetic. FIG. 4B is a graph showing the reduction in phlebotomy treatment following treatment with Compound 25.
FIGs. 5A and 5B provide graphs showing TSAT levels in HH patients over time following treatment with a hepcidin mimetic. FIG. 5A shows TSAT levels over the course of treatment for individual patients. For FIG. 5B, in the left graph, baseline transferrin saturation (%) is 45.0000 and post Compound 25 is 30.3948. In the right graph, baseline transferrin saturation (%) is 45.0000 and post Compound 25 is 36.2500.
FIG. 6 provides graphs showing serum iron levels in HH patients pre-treatment and the average serum iron levels following treatment with a hepcidin mimetic. In the right graph, baseline is 25.5454 and post Compound 25 is 17.6591.
FIG. 7 provides graphs showing dose- and concentration-dependent decreases in serum iron and TSAT following treatment with a hepcidin mimetic.
FIG. 8 provides graphs showing serum ferritin and serum transferrin levels in HH patients pretreatment and following treatment with a hepcidin mimetic. In the top left graph, baseline is 82.3431 and post Compound 25 is 85.0126. In the bottom left graph, baseline is 82.3431 and post Compound 25 is 114.4775. In the top right graph, baseline is 2.2869 and post Compound 25 is 2.4531. In the bottom right graph, baseline is 2.2869 and post Compound 25 is 2.4169.
FIG. 9 provides graphs showing liver iron content in HH patients pretreatment and following treatment with a hepcidin mimetic.
FIG. 10 is a summary of HH Patient Reported Outcomes following treatment with a hepcidin mimetic. The innermost point at Role Emotional and Role Physical correspond to screening, while the outermost point at Role Emotional and Role Physical correspond to post compound 25 treatment.
FIG. 11 is a diagram showing the Compound 25 dosages administered to each subject.
FIG. 12 is a graph showing transferrin saturation (%) over the course of six days following administration of the indicated dosages of Compound 25.
FIG. 13 includes graphs showing TST (%) and MCHC (g/dL) at baseline and 24 weeks following administration of Compound 25 to HH patients, and at baseline and after 4 weeks and 8 weeks in β-thalassemia transfusion dependent secondary iron overload patients.
FIG. 14 includes graphs showing results in HH patients following six months of treatment with Compound 25.

### DETAILED DESCRIPTION

The present disclosure identifies therapeutically effective dosages and dosing regimens of hepcidin mimetics useful for treating diseases and disorders associated with iron dysregulation, such as hereditary hemochromatosis (HH). In particular embodiments, the methods disclosed herein are practiced using Compound 25 to treat hereditary hemochromatosis.

### Definitions and Nomenclature

Unless otherwise defined herein, scientific and technical terms used in this application shall have the meanings that are commonly understood by those of ordinary skill in the art. Generally, nomenclature used in connection with, and techniques of, chemistry, molecular biology, cell and cancer biology, immunology, microbiology, pharmacology, and protein and nucleic acid chemistry, described herein, are those well-known and commonly used in the art.

As used herein, the following terms have the meanings ascribed to them unless specified otherwise.

Throughout this specification, the word "comprise" or variations such as "comprises" or "comprising" will be understood to imply the inclusion of a stated integer (or components) or group of integers (or components), but not the exclusion of any other integer (or components) or group of integers (or components).

The singular forms "a," "an," and "the" include the plurals unless the context clearly dictates otherwise.

The term "including" is used to mean "including but not limited to." "Including" and "including but not limited to" are used interchangeably.

The terms "patient," "subject," and "individual" may be used interchangeably and refer to either a human or a non-human animal. These terms include mammals such as humans, primates, livestock animals (e.g., bovines, porcines), companion animals (e.g., canines, felines) and rodents (e.g., mice and rats). The term "mammal" refers to any mammalian species such as a human, mouse, rat, dog, cat, hamster, guinea pig, rabbit, livestock, and the like.

The term "peptide," as used herein, refers broadly to a sequence of two or more amino acids joined together by peptide bonds. It should be understood that this term does not connote a specific length of a polymer of amino acids, nor is it intended to imply or distinguish whether the polypeptide is produced using recombinant techniques, chemical or enzymatic synthesis, or is naturally occurring.

The term "hepcidin mimetic," as used herein, refers broadly to peptide monomers and peptide dimers comprising one or more structural features and/or functional activities in common with hepcidin, or a functional region thereof. In certain embodiments, a hepcidin mimetic includes peptides sharing substantial amino acid sequence identity with hepcidin, e.g., peptides that comprise one or more amino acid insertions, deletions, or substitutions as compared to a wild-type hepcidin, e.g., human hepcidin, amino acid sequence. In certain embodiments, a hepcidin mimetic comprises one or more additional modification, such as, e.g., conjugation to another compound. Encompassed by the term "hepcidin mimetic" is any peptide monomer or peptide dimer disclosed herein. In some embodiments, a hepcidin mimetic has one or more functional activities of hepcidin.

The term "amino acid" or "any amino acid" as used here refers to any and all amino acids, including naturally occurring amino acids (e.g., a-amino acids), unnatural amino acids, modified amino acids, and non-natural amino acids. It includes both D- and L-amino acids. Natural amino acids include those found in nature, such as, e.g., the 23 amino acids that combine into peptide chains to form the building-blocks of a vast array of proteins. These are primarily L stereoisomers, although a few D-amino acids occur in bacterial envelopes and some antibiotics. The "non-standard," natural amino acids are pyrrolysine (found in methanogenic organisms and other eukaryotes), selenocysteine (present in many noneukaryotes as well as most eukaryotes), and N-formylmethionine (encoded by the start codon AUG in bacteria, mitochondria and chloroplasts). "Unnatural" or "non-natural" amino acids are non-proteinogenic amino acids (i.e., those not naturally encoded or found in the genetic code) that either occur naturally or are chemically synthesized. Over 140 natural amino acids are known and thousands of more combinations are possible. Examples of "unnatural" amino acids include β-amino acids (β³ and β²), homo-amino acids, proline and pyruvic acid derivatives, 3-substituted alanine derivatives, glycine derivatives, ring-substituted phenylalanine and tyrosine derivatives, linear core amino acids, diamino acids, D-amino acids, and N-methyl amino acids. Unnatural or non-natural amino acids also include modified amino acids. "Modified" amino acids include amino acids (e.g., natural amino acids) that have been chemically modified to include a group, groups, or chemical moiety not naturally present on the amino acid.

As is clear to the skilled artisan, the peptide sequences disclosed herein are shown proceeding from left to right, with the left end of the sequence being the N-terminus of the peptide and the right end of the sequence being the C-terminus of the peptide. Among sequences disclosed herein are sequences incorporating a "Hy-" moiety at the amino terminus (N-terminus) of the sequence, and either an "-OH" moiety or an "-NH₂" moiety at the carboxy terminus (C-terminus) of the sequence. In such cases, and unless otherwise indicated, a "Hy-" moiety at the N-terminus of the sequence in question indicates a hydrogen atom, corresponding to the presence of a free primary or secondary amino group at the N-terminus, while an "-OH" or an "-NH₂" moiety at the C-terminus of the sequence indicates a hydroxy group or an amino group, corresponding to the presence of an amido (CONH₂) group at the C-terminus, respectively. In each sequence of the invention, a C-terminal "-OH" moiety may be substituted for a C-terminal "-NH₂" moiety, and vice-versa. It is further understood that the moiety at the amino terminus or carboxy terminus may be a bond, e.g., a covalent bond, particularly in situations where the amino terminus or carboxy terminus is bound to a linker or to another chemical moiety, e.g., a PEG moiety.

The term "NH₂," as used herein, refers to the free amino group present at the amino terminus of a polypeptide. The term "OH," as used herein, refers to the free carboxy group present at the carboxy terminus of a peptide. Further, the term "Ac," as used herein, refers to Acetyl protection through acylation of the C- or N-terminus of a polypeptide.

The term "carboxy," as used herein, refers to -CO₂H.

For the most part, the names of naturally occurring and non-naturally occurring aminoacyl residues used herein follow the naming conventions suggested by the IUPAC Commission on the Nomenclature of Organic Chemistry and the IUPAC-IUB Commission on Biochemical Nomenclature as set out in "Nomenclature of α-Amino Acids (Recommendations, 1974)" Biochemistry, 14(2), (1975). To the extent that the names and abbreviations of amino acids and aminoacyl residues employed in this specification and appended claims differ from those suggestions, they will be made clear to the reader. Some abbreviations useful in describing the invention are defined below in the following Table 1.

**Table 1. Abbreviations of Non-Natural Amino Acids and Chemical Moieties**

| Abbreviation | Definition |
|---|---|
| DIG | Diglycolic acid |
| Dapa | Diaminopropionic acid |
| Daba | Diaminobutyric acid |
| Pen | Penicillamine |
| Sarc or Sar | Sarcosine |
| Cit | Citroline |
| Cav | Cavanine |
| NMe-Arg | N-Methyl-Arginine |
| NMe-Trp | N-Methyl-Tryptophan |
| NMe-Phe | N-Methyl-Phenylalanine |
| Ac- | Acetyl |
| 2-Nal | 2-Napthylalanine |
| 1-Nal | 1-Napthylalanine |
| Bip | Biphenylalanine |
| βAla | beta-Alanine |
| Aib | 2-aminoisobutyric acid |
| Azt | *azetidine-*2-carboxylic acid |
| Tic | (3S)-1,2,3,4-Tetrahydroisoquinoline-hydroxy-3-carboxylic acid |
| Phe(OMe) | Tyrosine (4-Methyl) |
| N-MeLys | N-Methyl-Lysine |
| N-MeLys(Ac) | N-e-Acetyl-D-lysine |
| Dpa | β,β diphenylalanine |
| NH₂ | Free Amine |
| CONH₂ | Amide |
| COOH | Acid |
| Phe(4-F) | 4-Fluoro-Phenylalanine |
| PEG3 | NH₂CH₂CH₂(OCH₂CH₂)₃CH₂CH₂CO₂H |
| m-PEG3 | CH₃OCH₂CH₂(OCH₂CH₂)₂CH₂CH₂CO₂H |
| m-PEG4 | CH₃OCH₂CH₂(OCH₂CH₂)₃CH₂CH₂CO₂H |
| m-PEG8 | CH₃OCH₂CH₂(OCH₂CH₂)₇CH₂CH₂CO₂H |
| PEG11 | O-(2-aminoethyl)-O'-(2-carboxyethyl)-undecaethyleneglycol NH₂CH₂CH₂(OCH₂CH₂)₁₁CH₂CH₂CO₂H |
| PEG13 | Bifunctional PEG linker with 13 PolyEthylene Glycol units |
| PEG25 | Bifunctional PEG linker with 25 PolyEthylene Glycol units |
| PEG1K | Bifunctional PEG linker with PolyEthylene Glycol Mol wt of 1000Da |
| PEG2K | Bifunctional PEG linker with PolyEthylene Glycol Mol wt of 2000Da |
| PEG3.4K | Bifunctional PEG linker with PolyEthylene Glycol Mol wt of 3400Da |
| PEG5K | Bifunctional PEG linker with PolyEthylene Glycol Mol wt of 5000Da |
| IDA or Ida | Iminodiacetic acid |
| IDA-Palm | (Palmityl)-Iminodiacetic acid |
| hPhe | homoPhenylalanine |
| Ahx | Aminohexanoic acid |
| DIG-OH | Glycolic monoacid |
| Triazine | Amino propyl Triazine di-acid |
| Boc-Triazine | Boc-Triazine di-acid |
| Trifluorobutyric acid | 4,4,4-Trifluorobutyric acid |
| 2-Methylltrifluorobutyric acid | 2-methyl-4,4,4-Butyric acid |
| Trifluorpentanoic acid | 5,5,5-Trifluoropentanoic acid |
| *1,4*- Phenylenediacetic acid | *para*-Phenylenediacetic acid |
| 1,3 - Phenylenediacetic acid | *meta*-Phenylenediacetic acid |
| DTT | Dithiothreotol |
| Nle | Norleucine |
| βhTrp or bhTrp | β-homoTryptophane |
| βhPhe or bhPhe | β-homophenylalanine |
| Phe(4-CF₃) | 4-TrifluoromethylPhenylalanine |
| βGlu or bGlu | β-Glutamic acid |
| βhGlu or bhGlu | β-homoglutamic acid |
| 2-2-Indane | 2-Aminoindane-2-carboxylic acid |
| 1-1-Indane | 1-Aminoindane-1-carboxylic acid |
| hCha | homocyclohexylalanine |
| Cyclobutyl | Cyclobutylalanine |
| hLeu | Homoleucine |
| Gla | γ-Carboxy-glutamic acid |
| Aep | 3-(2-aminoethoxy)propanoic acid |
| Aea | (2-aminoethoxy)acetic acid |
| IsoGlu-octanoic acid | octanoyl-γ-Glu |
| K-octanoic acid | octanoyl-ε-Lys |
| Dapa(Palm) | Hexadecanoyl-β-Diaminopropionic acid |
| IsoGlu-Palm | hexadecanoyl-γ-Glu |
| C-StBu | S-tert-butylthio-cysteine |
| C-tBu | S-tert-butyl-cysteine |
| Dapa(AcBr) | NY-(bromoacetyl)-2,3- diaminopropionic acid |
| Tle | *tert*-Leucine |
| Phg | phenylglycine |
| Oic | octahydroindole-2-carboxylic acid |
| Chg | α-cyclohexylglycine |
| GP-(Hyp) | Gly-Pro-HydroxyPro |
| Inp | isonipecotic acid |
| Amc | 4-(aminomethyl)cyclohexane carboxylic acid |
| Betaine | (CH₃)₃NCH₂CH₂CO2H |
| D-Npc | Isonipecotic acid |
| Npc | Nipecotic acid |

Throughout the present specification, unless naturally occurring amino acids are referred to by their full name (e.g., alanine, arginine, etc.), they are designated by their conventional three-letter or single-letter abbreviations (e.g., Ala or A for alanine, Arg or R for arginine, etc.). In the case of less common or non-naturally occurring amino acids, unless they are referred to by their full name (e.g. sarcosine, ornithine, etc.), frequently employed three- or four-character codes are employed for residues thereof, including, Sar or Sarc (sarcosine, i.e., N-methylglycine), Aib (α-aminoisobutyric acid), Daba (2,4-diaminobutanoic acid), Dapa (2,3-diaminopropanoic acid), γ-Glu (y-glutamic acid), pGlu (pyroglutamic acid), Gaba (y-aminobutanoic acid), β-Pro (pyrrolidine-3-carboxylic acid), 8Ado (8-amino-3,6-dioxaoctanoic acid), Abu (4-aminobutyric acid), bhPro (β-homo-proline), bhPhe (β-homo-L-phenylalanine), bhAsp (β-homo-aspartic acid]), Dpa (β,β diphenylalanine), Ida (Iminodiacetic acid), hCys (homocysteine), and bhDpa (β-homo-β,β -diphenylalanine).

Furthermore, R1 can in all sequences be substituted with isovaleric acids or equivalent. In some embodiments, wherein a peptide of the present invention is conjugated to an acidic compound such as, e.g., isovaleric acid, isobutyric acid, valeric acid, and the like, the presence of such a conjugation is referenced in the acid form. So, for example, but not to be limited in any way, instead of indicating a conjugation of isovaleric acid to a peptide by referencing isovaleroyl, in some embodiments, the present application may reference such a conjugation as isovaleric acid.

The term "L-amino acid," as used herein, refers to the "L" isomeric form of a peptide, and conversely the term "D-amino acid" refers to the "D" isomeric form of a peptide. In certain embodiments, the amino acid residues described herein are in the "L" isomeric form, however, residues in the "D" isomeric form can be substituted for any L-amino acid residue, as long as the desired functional is retained by the peptide.

Unless otherwise indicated, reference is made to the L-isomeric forms of the natural and unnatural amino acids in question possessing a chiral center. Where appropriate, the D-isomeric form of an amino acid is indicated in the conventional manner by the prefix "D" before the conventional three-letter code (e.g., Dasp, (D)Asp or D-Asp; Dphe, (D)Phe or D-Phe).

The term "dimer," as used herein, refers broadly to a peptide comprising two or more monomer subunits. Certain dimers comprise two DRPs. Dimers of the present invention include homodimers and heterodimers. A monomer subunit of a dimer may be linked at its Cor N-terminus, or it may be linked via internal amino acid residues. Each monomer subunit of a dimer may be linked through the same site, or each may be linked through a different site (e.g., C-terminus, N-terminus, or internal site).

As used herein, in the context of certain peptide sequences disclosed herein, parentheticals, e.g., (__), represent side chain conjugations and brackets, e.g., [__], represent unnatural amino acid substitutions or amino acids and conjugated side chains. Generally, where a linker is shown at the N-terminus of a peptide sequence, it indicates that the peptide is dimerized with another peptide, wherein the linker is attached to the N-terminus of the two peptides. Generally, where a linker is shown at the C-terminus of a peptide sequence or structure, it indicates that the peptide is dimerized with another peptide, wherein the linker is attached to the C-terminus of the two peptides.

The term "cyclized," as used herein, refers to a reaction in which one part of a polypeptide molecule becomes linked to another part of the polypeptide molecule to form a closed ring, such as by forming a disulfide bridge or other similar bond.

The term "subunit," as used herein, refers to one of a pair of polypeptide monomers that are joined to form a dimer peptide composition.

The term "linker moiety," as used herein, refers broadly to a chemical structure that is capable of linking or joining together two peptide monomer subunits to form a dimer.

The term "solvate" in the context of the present invention refers to a complex of defined stoichiometry formed between a solute (e.g., a hepcidin analogue or pharmaceutically acceptable salt thereof according to the invention) and a solvent. The solvent in this connection may, for example, be water, ethanol or another pharmaceutically acceptable, typically small-molecular organic species, such as, but not limited to, acetic acid or lactic acid. When the solvent in question is water, such a solvate is normally referred to as a hydrate.

The term "pharmaceutically acceptable salt," as used herein, represents salts or zwitterionic forms of the peptides or compounds of the present invention which are water or oil-soluble or dispersible, which are suitable for treatment of diseases without undue toxicity, irritation, and allergic response; which are commensurate with a reasonable benefit/risk ratio, and which are effective for their intended use. The salts can be prepared during the final isolation and purification of the compounds or separately by reacting an amino group with a suitable acid. Representative acid addition salts include acetate, adipate, alginate, citrate, aspartate, benzoate, benzenesulfonate, bisulfate, butyrate, camphorate, camphorsulfonate, digluconate, glycerophosphate, hemisulfate, heptanoate, hexanoate, formate, fumarate, hydrochloride, hydrobromide, hydroiodide, 2-hydroxyethansulfonate (isethionate), lactate, maleate, mesitylenesulfonate, methanesulfonate, naphthylenesulfonate, nicotinate, 2-naphthalenesulfonate, oxalate, pamoate, pectinate, persulfate, 3-phenylproprionate, picrate, pivalate, propionate, succinate, tartrate, trichloroacetate, trifluoroacetate, phosphate, glutamate, bicarbonate, para-toluenesulfonate, and undecanoate. Also, amino groups in the compounds of the present invention can be quaternized with methyl, ethyl, propyl, and butyl chlorides, bromides, and iodides: dimethyl, diethyl, dibutyl, and diamyl sulfates; decyl, lauryl, myristyl, and steryl chlorides, bromides, and iodides; and benzyl and phenethyl bromides. Examples of acids which can be employed to form therapeutically acceptable addition salts include inorganic acids such as hydrochloric, hydrobromic, sulfuric, and phosphoric, and organic acids such as oxalic, maleic, succinic, and citric. A pharmaceutically acceptable salt may suitably be a salt chosen, e.g., among acid addition salts and basic salts. Examples of acid addition salts include chloride salts, citrate salts and acetate salts. Examples of basic salts include salts where the cation is selected among alkali metal cations, such as sodium or potassium ions, alkaline earth metal cations, such as calcium or magnesium ions, as well as substituted ammonium ions, such as ions of the type N(R1)(R2)(R3)(R4)+, where R1, R2, R3 and R4 independently will typically designate hydrogen, optionally substituted C1-6-alkyl or optionally substituted C2-6-alkenyl. Examples of relevant C1-6-alkyl groups include methyl, ethyl, 1-propyl and 2-propyl groups. Examples of C2-6-alkenyl groups of possible relevance include ethenyl, 1-propenyl and 2-propenyl. Other examples of pharmaceutically acceptable salts are described in "Remington's Pharmaceutical Sciences", 17th edition, Alfonso R. Gennaro (Ed.), Mark Publishing Company, Easton, PA, USA, 1985 (and more recent editions thereof), in the "Encyclopaedia of Pharmaceutical Technology", 3rd edition, James Swarbrick (Ed.), Informa Healthcare USA (Inc.), NY, USA, 2007, and in J. Pharm. Sci. 66: 2 (1977). Also, for a review on suitable salts, see Handbook of Pharmaceutical Salts: Properties, Selection, and Use by Stahl and Wermuth (Wiley-VCH, 2002). Other suitable base salts are formed from bases which form non-toxic salts. Representative examples include the aluminum, arginine, benzathine, calcium, choline, diethylamine, diolamine, glycine, lysine, magnesium, meglumine, olamine, potassium, sodium, tromethamine, and zinc salts. Hemisalts of acids and bases may also be formed, e.g., hemisulphate and hemicalcium salts.

The term "alkyl" includes a straight chain or branched, noncyclic or cyclic, saturated aliphatic hydrocarbon containing from 1 to 24 carbon atoms. Representative saturated straight chain alkyls include, but are not limited to, methyl, ethyl, *n*-propyl, *n*-butyl, *n*-pentyl, *n*-hexyl, and the like, while saturated branched alkyls include, without limitation, isopropyl, sec-butyl, isobutyl, *tert-*butyl*,* isopentyl, and the like. Representative saturated cyclic alkyls include, but are not limited to, cyclopropyl, cyclobutyl, cyclopentyl, cyclohexyl, and the like, while unsaturated cyclic alkyls include, without limitation, cyclopentenyl, cyclohexenyl, and the like.

As used herein, a "therapeutically effective amount" of the peptide agonists of the invention is meant to describe a sufficient amount of the peptide agonist to treat an hepcidin-related disease, including but not limited to any of the diseases and disorders described herein (for example, a disease of iron metabolism). In particular embodiments, the therapeutically effective amount will achieve a desired benefit/risk ratio applicable to any medical treatment.

### Methods for Using Hepcidin Mimetics

Hepcidin targets the major iron transporter ferroportin and causes its internalization and subsequent degradation. Hepcidin regulation is crucial for providing adequate iron needed for cellular functions while also preventing iron toxicity. In Hereditary Hemochromatosis (HH), hyperabsorption of dietary iron leads to primary iron overload. Under these iron overload conditions, where transferrin is saturated (e.g., Transferrin SATuration (TSAT) % > 80%), excess iron deposition can lead to organ damage. In addition, the presence of labile iron increases overall systemic iron toxicity. The present disclosure identifies methods of dosing and using hepcidin mimetics with a therapeutic effect for the treatment of diseases and disorders associated with iron overload, such as hereditary hemochromatosis.

In some embodiments, methods disclosed herein are applied for the prevention, inhibition, or treatment of a disease or disorder associated with dysregulated iron levels (e.g., diseases or disorders of iron metabolism; diseases or disorders related to iron overload; and diseases or disorders related to abnormal hepcidin activity or expression). In certain embodiments, the disease or disorder is a disease of iron metabolism, such as, e.g., an iron overload disease or another disorder of iron metabolism.

In particular embodiments, the disease of iron metabolism is a hemochromatosis, such as, e.g., hereditary hemochromatosis, HFE mutation hemochromatosis, ferroportin mutation hemochromatosis, transferrin receptor 2 mutation hemochromatosis, hemojuvelin mutation hemochromatosis, hepcidin mutation hemochromatosis, juvenile hemochromatosis, or neonatal hemochromatosis. In certain embodiments, the disease is hereditary hemochromatosis (HH). In certain embodiments, the disease is phlebotomy-requiring HH, e.g., HH in the maintenance phase.

In certain embodiments, the disease or disorder is HH associated with arthropathy, or HH-associated arthropathy. Chronic arthropathy occurs in 37-80% of HH patients. In these cases, joint pain may be an early manifestation of the disease and in many cases is the cause for first diagnosis of HH. This may be done by X-ray and/or MRI imaging, often combined with validated joint pain and/or results of function scoring instruments. In certain embodiments, the arthropathy may be associated with elevated age, ferritin, and TSAT levels. The iron accumulation of HH may be associated with increased oxidative stress, disrupted matrix metabolism, and cartilage degeneration, which can contribute to the development of arthropathy similar to osteoarthritis. Persistent arthropathy can diminish quality of life and yield high healthcare utilization and associated costs, particularly since up to 16% of HH patients undergo joint replacement surgery. See, e.g., Whalen, N. "Association of Transferrin Saturation with the Arthropathy of Hereditary Hemochromatosis" 2017: Nguyen, C. "Bone and joint complications in patients with hereditary hemochromatosis: a cross-sectional study of 93 patients" 2020; Carroll, GJ. "Hereditary Hemochromatosis is characterized by a clinically definable arthropathy that correlates with iron load" 2011: Karim, A. "The role of disrupted iron homeostasis in the development and progression of arthropathy" 2022; and Burton, LH. "Systemic administration of a pharmacologic iron chelator reduces cartilage lesion development in the Dunkin-Hartley model of primary osteoarthritis" 2022.

In one aspect, the disclosure provides a method for treating an iron overload disease, e.g., HH, HH associated with arthropathy, or HH-associated arthropathy, in a human subject, comprising providing to the subject an effective amount of a hepcidin mimetic, including but not limited to those disclosed herein, such as Compound 25. In certain embodiments, the hepcidin mimetic is provided subcutaneously. In certain embodiments, the subject has been diagnosed with hereditary hemochromatosis. In certain embodiments, the subject is receiving or requires phlebotomy treatment prior to the treatment disclosed herein. In particular embodiments, prior to treatment with a hepcidin mimetic according to the disclosed methods, the subject was receiving at least three, at least four, at least five, or at least six phlebotomies per year. In certain embodiments, the subject was receiving about five to about six phlebotomies per year. In particular embodiments, the subject's HH is in the maintenance phase, and the subject is being treated by phlebotomy less than once per week, for example, about once per month, or about once every two to four months. In certain embodiments, the subject has confirmed HH and was on stable phlebotomies for at least three months or at least six months prior to treatment according to the disclosure with a phlebotomy frequency of about 0.25 to 1 per month. In certain embodiments, the subject does not have clinically meaningful laboratory abnormalities and/or is not receiving iron chelation therapy or erythrocytapheresis. In particular embodiments, the patient has difficult with phlebotomy, e.g., due to issues with venous access resulting from accumulated needle sticks due to multiple phlebotomies over time. In certain embodiments, the subject has iron-deficient anemia, and in certain embodiments, the subject is needle phobic.

In certain embodiments, the effective amount of the hepcidin mimetic, e.g., Compound 25, is about 1 mg to about 100 mg, or about 10 mg to about 80 mg. In certain embodiments, the effective amount of the hepcidin mimetic, e.g., Compound 25, is about 5 mg to about 80 mg, or about 10 mg to about 80 mg. In certain embodiments, the effective amount of the hepcidin mimetic, e.g., Compound 25, is about 10 mg to about 40 mg, or about 5 mg to about 50 mg. In certain embodiments, the effective amount of the hepcidin mimetic, e.g., Compound 25, is about 10 mg to about 40 mg, or about 5 mg to about 50 mg. In particular embodiments, the effective amount of the hepcidin mimetic is administered to the subject about once a week or about twice a week. In some embodiments, the subject is administered about 10 mg to about 20 mg, about 10 mg to about 15 mg, or about 15 mg to about 20 mg, of the hepcidin mimetic about twice a week, and in another embodiments, the subject is administered about 20 mg to about 30 mg, about 20 mg to about 40 mg, or about 30 mg to about 40 mg of the hepcidin mimetic about once a week. In certain embodiments, the subject is administered about 5 mg, about 6 mg, about 7 mg, about 8 mg, about 9 mg, about 10 mg, about 11 mg, about 12 mg, about 13 mg, about 14 mg, about 15 mg, about 16 mg, about 17 mg, about 18 mg, about 19 mg, about 20 mg, about 21 mg, about 22 mg, about 23 mg, about 24 mg, or about 25 mg of the hepcidin mimetic about twice a week. In certain embodiments, the subject is administered about 15 mg, about 16 mg, about 17 mg, about 18 mg, about 19 mg, about 20 mg, about 21 mg, about 22 mg, about 23 mg, about 24 mg, about 25 mg, about 26 mg, about 27 mg, about 28 mg, about 29 mg, about 30 mg, about 31 mg, about 32 mg, about 33 mg, about 34 mg, about 35 mg, about 36 mg, about 37 mg, about 38 mg, about 39 mg, about 40 mg, about 41 mg, about 42 mg, about 43 mg, about 44 mg, or about 45 mg of the hepcidin mimetic about twice a week. In particular embodiments, the hepcidin mimetic is administered subcutaneously. In particular embodiments, the hepcidin mimetic is Compound 25. In certain embodiments, the amount administered to the subject may vary over the course of treatment.

In embodiments, the methods reduce transferrin saturation (TSAT) level and/or serum iron level and/or mean corpuscular hemoglobin concentration (MCHC) level in the subject by at least 30%, at least 40%, or at least 60%. In some embodiments, the effective amount causes a decrease in the subject's transferrin saturation (TSAT) level and/or serum iron of at least 30%, at least 40%, at least 50%, at least 60%, at least 70%, at least 80%, or at least 90%. In certain embodiments, the TSAT% and/or serum iron level and/or MCHC level is reduced between about 40% and about 90%, between about 50% and about 90%, or between 50% and 75%. In some embodiments, TSAT% level and/or serum iron level and/or MCHC level is reduced to a level less than 90%, less than 80%, less than 70%, less than 60%, or less than 50% of the level observed in normal, healthy volunteers of the same mammal type. In particular embodiments, the reduction in the TSAT% level and/or serum iron level and/or MCHC level is the maximum reduction observed in the subject following treatment with the agent, while in other embodiments, the reduction in the TSAT% level and/or serum iron level is the reduction observed at trough level of the agent following administration to a subject. In some embodiments, the reduction in the TSAT% level and/or serum iron level and/or MCHC level is the average reduction observed, e.g., in a plurality of subjects.

In some embodiments, the subject's TSAT level is reduced to ≤ 60% TSAT, ≤ 50% TSAT, ≤ 45% TSAT, ≤ 40% TSAT, ≤ 30% TSAT, ≤ 20% TSAT, or ≤ 10% TSAT. In particular embodiments, the subject's TSAT level is reduced to ≤ 45% TSAT, or ≤ 40% TSAT. In particular embodiments, the subject's TSAT level is reduced to ≤ 40% TSAT, or ≤ 35% TSAT. In certain embodiments, the TSAT level is reduced to between about 20% TSAT and about 50% TSAT, e.g., between about 25% TSAT to about 40% TSAT. In particular embodiments, the TSAT% and/or serum iron level is reduced by at least 10%, at least 20%, at least 40%, at least 40%, at least 50%, at least 60%, at least 70%, or at least 90%. In certain embodiments, the TSAT% value is reduced to about 0% to about 60%, about 0% to about 40%, about 10% to about 40%, about 10% to about 30%, about 10% to about 20%, about 20% to about 60%, about 20% to about 40%, or about 20% to about 30%. In some embodiments, TSAT% is reduced to less than or equal to 50% TSAT, less than or equal to 45% TSAT, less than or equal to 40% TSAT, less than or equal to 35% TSAT, less than or equal to 30% TSAT, less than or equal to 20% TSAT, less than or equal to 15% TSAT, less than or equal to 10% TSAT, between about 10% TSAT and about 45% TSAT, between about 5% TSAT and about 20% TSAT, or between about 10% TSAT and about 20% TSAT at the time tested. In some embodiments, TSAT% level and/or serum iron level is reduced to a level less than 90%, less than 80%, less than 70%, less than 60%, or less than 50% of the level observed in normal, healthy volunteers of the same mammal type.

In certain embodiments, the serum iron level is reduced to about 0 uM to about 30 uM, about 0 uM to about 5 uM, about 0 uM to about 20 uM, about 0 uM to about 15 uM, about 0 uM to about 10 uM, or about 0 uM to about 5 uM. In certain embodiments, the subject's serum iron level is reduced to ≤ 20 umol/L, ≤ 18 umol/L, ≤ 16 umol/L, ≤ 14 umol/L, ≤ 12 umol/L, ≤ 10 umol/L, ≤ 8 umol/L, ≤ 6 umol/L, or ≤ 4 umol/L. In some embodiments, the subject's serum iron level is reduced to between about 2 umol/L and about 100 umol/L, e.g., between 2 and about 10 umol/L, or between about 10 umol/L and about 50 umol/L, or between about 10 umol/L and 30 about umol/L. In some embodiments, the subject's serum iron level is reduced to between about 2 umol/L and about 100 umol/L, e.g., between 2 and about 10 umol/L, or between about 10 umol/L and about 50 umol/L, or between about 10 umol/L and 30 about umol/L. In some embodiments, the subject's serum iron level is reduced to between about 50 mcg/dL to about 200 mcg/dL, or about 60 mcg/dL to about 170 mcg/dL. In certain embodiments, the serum iron level is reduced to less than about 150 ug/dL, less than about 100 ug/dL, or less than about 75 ug/dL.

In some embodiments, the subject's MCHC level is reduced by at least 5%, at least 10%, at least 20%, at least 40%, at least 40%, at least 50%, at least 60%, at least 70%, or at least 90%. In certain embodiments, the MCHC value (g/dL) is reduced to less than about 35, less than or about 34, less than or about 33, less than or about 32, less than or about 31, less than or about 30, or less than or about 29.

In embodiments, the methods reduce transferrin saturation (TSAT) level and/or serum iron and/or MCHC level in the subject by at least 10%, at least 20%, at least 30%, at least 40%, or at least 60%. In some embodiments, the effective amount causes a decrease in the subject's transferrin saturation (TSAT) level and/or serum iron and/or MCHC level of at least 30%, at least 40%, at least 50%, at least 60%, at least 70%, at least 80%, or at least 90%. In certain embodiments, the TSAT% and/or serum iron level and/or MCHC level is reduced between about 40% and about 90%, between about 50% and about 90%, or between 50% and 75%. In some embodiments, TSAT% level and/or serum iron level and/or MCHC level is reduced to a level less than 90%, less than 80%, less than 70%, less than 60%, or less than 50% of the level observed in normal, healthy volunteers of the same mammal type. In particular embodiments, the reduction in the TSAT% level and/or serum iron level and/or MCHC level is the maximum reduction observed in the subject following treatment with the agent, while in other embodiments, the reduction in the TSAT% level and/or serum iron level and/or MCHC level is the reduction observed at trough level of the agent following administration to a subject. In some embodiments, the reduction in the TSAT% level and/or serum iron level and/or MCHC level is the average reduction observed, e.g., in a plurality of subjects.

In some embodiments, TSAT% level and/or serum iron level and/or MCHC level is reduced to a level less than 200%, less than 150%, less than 100T, less than 90%, less than 80%, less than 70%, less than 60%, or less than 50% of the level observed in normal, healthy volunteers of the same mammal type.

In certain embodiments, following treatment with a hepcidin mimetic, e.g., Compound 25, an HH patient exhibits: TSAT levels of ≤ 45% or ≤ 40%, serum iron levels of < 150 ug/dL, < 100 ug/dL, or < 75 ug/dL; and/or MCHC levels of < 35 g/dL or < 30 g/dL. In particular embodiments, these levels occur at trough concentration of the hepcidin mimetic, e.g., about 7 days following the last dosing.

In certain embodiments, the decrease in the subject's transferrin saturation (TSAT) level and/or serum iron level and/or MCHC level occurs for at least one day, at least two days, at least three days, at least four days, at least five days, at least six days, or at least one week following administration of the hepcidin mimetic. In particular embodiments, the subject is provided with the hepcidin mimetic about twice a week, or about once a week, e.g., as a single dose or over a period of time. In certain embodiments, the decrease in the subject's transferrin saturation (TSAT) level and/or serum iron level and/or MCHC level is at least 50%, or at least 60% or at least 80% for at least one day, at least two days, at least 3 days, or at least 4 days following a dose. In particular embodiments, the decrease in TSAT and/or serum iron level and/or MCHC is maintained over the course of treatment, wherein the course of treatment may be, e.g., once a week or twice a week for at least two months, at least four months, at least six months, at least one year, at least two years, at least three years or longer. In certain embodiments, the dosing regimen for a subject may change throughout the course of treatment, but in particular embodiments, administration of the hepcidin mimetic (e.g., Compound 25) occurs about once a week or about twice a week over the course of treatment, and the dosage is about 5 mg to about 40 mg, or about 10 mg to about 40 mg, per administration. In particular embodiments, the subject is administered different dosages (at the same or different frequency), or the same dosage at different frequency, during different time periods of the course of treatment, but in particular embodiments, the frequency throughout the course of treatment is about once or twice per week, and each dosage administered is in the range of about 5 mg to about 40 mg. In some embodiments, the agent is a hepcidin mimetic peptide disclosed herein, e.g., a peptide of any one of Formula I-VIII or any of Compounds 1-34, such as Compound 25. In certain embodiments, the decrease in the subject's transferrin saturation (TSAT) level and/or serum iron level is at least 60% for at least one day.

In certain embodiments, the subject needs fewer phlebotomies following treatment with a hepcidin mimetic, e.g., Compounds 25, according to the disclosed methods. In some embodiments, the subject needs less than 0.1 phlebotomy per month, or less than 0.05 phlebotomy per month during treatment according to the disclosure.

In one embodiment, the method comprises treating a subject having HH, wherein prior to treatment the subject is on a stable phlebotomy frequency of 0.25 - 1.0 per month for at least six months, by administering to the subject an effective amount of a hepcidin mimetic, e.g., Compound 25. In particular embodiments, the subject is administered about 5 to about 20 mg (e.g., about 5 mg, about 10 mg, about 15 mg or about 20 mg) of the hepcidin mimetic about twice a week, and/or is administered about 10 to about 40 mg (e.g., about 15 mg, about 20 mg, about 25 mg, about 30 mg, about 35 mg, or about 40 mg) of the hepcidin mimetic about once a week. In particular embodiments, the subject is administered different dosages at different frequency during different time periods. In particular embodiments, the subject is administered the hepcidin mimetic for at least one month, at least two months, at least four months, at least six months, or at least one year. In particular embodiments, the subject's phlebotomy frequency is reduced over the course of treatment to less than 0.1 phlebotomy per month, or less than 0.05 phlebotomy per month.

In certain embodiments, the subject has improved arthropathy following treatment, e.g., as determined by methods available in the art, e.g., X-ray, MRI, joint pain, and/or use of functional scoring instruments.

In certain embodiments, the subject has reduced oxidative stress, reduced disrupted matrix metabolism, and/or reduced cartilage degeneration following treatment.

In certain embodiments, the methods disclosed herein result in the subject having decreased circulating transferrin saturation (TSAT) and/or decreased toxic non-transferrin bound iron (NTBI), and/or decreased iron accumulation in organs, e.g., such as the liver, pancreas, heart, and bone.

If untreated, iron overload can cause hepatomegaly, diabetes mellitus, skin hyperpigmentation, cardiomyopathy, diastolic dysfunction, heart failure, cirrhosis, etc. In particular embodiments, methods of treatment disclosed herein reduce, alleviate, or improve any of these symptoms or pathologies associated with iron overload, such as HH.

In certain embodiments, the method further comprises determining a TSAT level and/or serum iron level and/or MCHC level in the subject before and/or after treatment with the hepcidin mimetic. In particular embodiments, the subject has a reduction in TSAT and/or serum iron and/or MCHC of at least 20%, at least 30%, at least 40%, at least 50%, at least 60% or at least 80%, and is provided with the agent. In certain embodiments, the method comprises measuring a TSAT% level in the subject before and/or after providing the subject with the agent, and in some embodiments, the method comprises providing additional agent to the subject to achieve or maintain the reduced TSAT% level, e.g., ≤ 45% TSAT or ≤ 40% TSAT. In particular embodiments, the agent is any of those disclosed herein, e.g., a hepcidin mimetic peptide, such as a peptide of any one of Formula I-VIII or any of Compounds 1-34, e.g., Compound 25.

In some embodiments, the method comprises determining TSAT% and/or serum iron levels and/or MCHC levels in a subject before and after administering the hepcidin mimetic to the subject, and then determining whether the subject's TSAT% and/or serum iron levels and/or MCHC levels were reduced to the desired level after administration of the hepcidin mimetic. In some embodiments, the subject is given increasing amounts of the hepcidin mimetic until TSAT% and/or serum iron levels and/or MCHC levels are reduced to the desired level. In some embodiments, the subject is given multiple different doses of the hepcidin mimetic over a course of treatment (and potentially at different frequencies, such as once a week and/or twice a week), and the subject's TSAT% and/or serum iron levels and/or MCHC levels are monitored at various times during a course of treatment to identify the minimal or appropriate dose and/or concentration required to reduce TSAT% and/or serum iron levels to the desired level, and/or to identify how frequently the subject should be treated to maintain the subject's TSAT% and/or serum iron levels and/or MCHC levels at the desired level. In certain embodiments, the method comprises measuring a TSAT% level in the subject before and/or after administering the hepcidin mimetic to the subject, and in some embodiments, the method comprises providing additional hepcidin mimetic to the subject to achieve or maintain the reduced TSAT% level, e.g., a level at or below 45% TSAT. In particular embodiments, the hepcidin mimetic is a peptide of any one of Formula I-VIII or any of Compounds 1-34, e.g., Compound 25.

In certain embodiments, the disclosure provides a method for treating HH, HH associated with arthropathy, or HH-associated arthropathy, comprising:
a) subcutaneously administering to a subject diagnosed with HH, e.g., phlebotomy-dependent HH, HH associated with arthropathy, or HH-associated arthropathy, about 5 to about 25 mg (optionally 10 mg or 20 mg) of a hepcidin mimetic disclosed herein, e.g., Compound 25;
b) determining the subject's TSAT % following step a), optionally at trough drug level, e.g., about 7 days following step a); and
c) if the subject's TSAT % is greater than about 40% or greater than about 45%,
   (i) subcutaneously administering to the subject an increased amount of the hepcidin mimetic, e.g., about 20 mg to about 80 mg, optionally about 20 mg or about 40 mg, on an about weekly or about twice weekly schedule; or
   (ii) subcutaneously administering to the subject the same or an increased amount of the hepcidin mimetic, e.g., about 10 mg or about 20 mg or about 40 mg, on an increased dosing schedule, e.g., about twice weekly.
In particular embodiments, the method comprises monitoring the subject's TSAT % levels, e.g., about 7 days after the first weekly dose, and adjusting the dosing by increasing the dose or increasing the frequency of dosing, if the TSAT% is greater than 40% or greater than 45%. The method may further include determining and/or monitoring the subject's serum iron level and/or MCHC level, and adjusting the dosage amount or frequency based on either or both.

In various embodiments of any of the methods disclosed herein, the TSAT% level and/or serum iron level and/or MCHC levels is reduced by a percentage, or it is reduced to or below a particular TSAT% level or serum iron level and/or MCHC levels, e.g., in order to be associated with a therapeutically effective agent or dosing regimen. In particular embodiments, the reduction in the TSAT% level and/or serum iron level and/or MCHC levels is maintained for a duration of time, e.g., 12 hours, one day, two days, three days, four days, five days, six days, or one week. As would be understood, a percentage reduction in any of these levels could be the percentage reduction in a particular patient, for example, when monitoring TSAT% level and/or serum iron level and/or MCHC levels to determine dosing or dosing regimen for the patient, or a percentage reduction in TSAT% level and/or serum iron level and/or MCHC levels could be a reduction as compared to a predetermined value, e.g., the average or mean TSAT% level, or serum iron level, or MCHC level associated with a particular patient population. In certain embodiments, the reduction in TSAT% is a reduction as compared to a normal, healthy volunteer.

Serum iron can be measured by various methods including colorimetrically. TSAT represents the percentage of the transferrin iron-binding capacity actually occupied by iron in the serum. It is calculated as the serum iron multiplied by 100 and divided by the total iron-binding capacity (Coyne. Kidney International 69:54-58).

### Hepcidin Mimetics

Methods disclosed herein may be practiced various hepcidin mimetics, including but not limited to those described herein, such as Compound 25. In certain embodiments, the agent modulates serum iron levels, e.g., by temporarily sequestering or redistributing iron in various tissues and/or preventing further absorption of iron from food. In some embodiments, an iron sequestration compound prevents export of iron. In various embodiments, the agent effects serum iron levels and/or iron load or distribution in various tissues/organs. It is understood that the disclosure further relates to pharmaceutically acceptable salts and solvates on any of the agents disclosed herein.

In particular embodiments, the hepcidin mimetic is described in any of the following: US patents, US 9,822,157 and US 10,030,061, which describe hepcidin analogs and their use to treat iron overload diseases, which include hereditary hemochromatosis and iron-loading anemias; PCT application publication, WO15200916, which describes additional hepcidin analogs and their use to treat iron overload diseases: PCT application publication, WO17117411, which describes additional hepcidin analogs with improved *in vivo* half-lives and their use to treat iron overload diseases; PCT application publication, WO18048944, which describes additional hepcidin analogs and their use to treat prevention of iron overload in a subject and/or reducing serum iron levels in a subject; PCT application publication, WO18128828, which describes additional hepcidin analogs and their use to treat hepcidin-associated disorders, including prophylaxis and treatment of iron overload diseases such as hemochromatosis, iron-loading anemias such as thalassemia, and diseases being associated with ineffective or augmented erythropoiesis; PCT application publication, WO17068089, which describes additional hepcidin analogs (ferroportin inhibitors) and their use to treat thalassemia and hemochromatosis; or US Patent, US9315545, which describe additional novel analogs and their use to treat diseases of iron metabolism, beta thalassemia, hemochromatosis, iron-loading anemias, alcoholic liver disease, or chronic hepatitis C.

In certain embodiments, the hepcidin mimetic is a peptide comprising or consisting of Formula I:

R1-X-Y-R2 (I)

or a pharmaceutically acceptable salt or solvate thereof,
wherein
R1 is hydrogen, a C1-C6 alkyl, a C6-C12 aryl, a C1-C20 alkanoyl, or pGlu;
R2 is NH₂ or OH;
X is an amino acid sequence of Formula II:

   X1-X2-X3-X4-X5-X6-X7-X8-X9-X10 (II)
wherein
X1 is Asp, Ala, Ida, pGlu, bhAsp, Leu, D-Asp, or absent;
X2 is Thr, Ala, or D-Thr:
X3 is His, Lys, D-His, or Lys:
X4 is Phe, Ala, Dpa, or D-Phe:
   X5 is Pro, Gly, Arg, Lys, Ala, D-Pro, or bhPro;
   X6 is Ile, Cys, Arg, Lys, D-Ile, or D-Cys;
   X7 is Cys, Ile, Leu, Val, Phe, D-Ile, or D-Cys;
   X8 is Ile, Arg, Phe, Gln, Lys, Glu, Val, Leu, or D-Ile;
   X9 is Phe or bhPhe; and
   X10 is Lys, Phe, or absent;
   wherein if Y is absent, X7 is Ile; and
   Y is an amino acid sequence of Formula III:

      Y1-Y2-Y3-Y4-Y5-Y6-Y7-Y8-Y9-Y10-Y11-Y12-Y13-Y14-Y15 (III)
   wherein
   Y1 is Gly, Cys, Ala, Phe, Pro, Glu, Lys, D-Pro, Val, Ser, or absent:
      Y2 is Pro, Ala, Cys, Gly, or absent;
      Y3 is Arg, Lys, Pro, Gly, His, Ala, Trp, or absent:
         Y4 is Ser, Arg, Gly, Trp, Ala, His, Tyr, or absent:
         Y5 is Lys, Met, Arg, Ala, or absent;
         Y6 is Gly, Ser, Lys, Ile, Ala, Pro, Val, or absent:
            Y7 is Trp, Lys, Gly, Ala, Ile, Val, or absent;
            Y8 is Val, Thr, Gly, Cys, Met, Tyr, Ala, Glu, Lys, Asp, Arg, or absent;
            Y9 is Cys, Tyr, or absent;
            Y10 is Met, Lys, Arg, Tyr, or absent;
            Y11 is Arg, Met, Cys, Lys, or absent;
            Y12 is Arg, Lys, Ala or absent;
            Y13 is Arg, Cys, Lys, Val or absent;
            Y14 is Arg, Lys, Pro, Cys, Thr or absent; and
            Y15 is Thr, Arg or absent:
               wherein the peptide comprising Formula I is optionally PEGylated on R1, X, or Y;
               wherein a side chain of an amino acid of the peptide is optionally conjugated to a lipophilic substituent or polymeric moiety; and
               wherein Ida is iminodiacetic acid, pGlu is pyroglutamic acid, bhAsp is β-homoaspartic acid, and bhPro is β-homoproline.

In certain embodiments, any of the peptides disclosed herein comprise a disulfide bond between two Cys amino acid residues present in the peptide, e.g., wherein the thiol groups of two cysteine residues in the peptide form a disulfide bond.

In certain embodiments, R1 is hydrogen, isovaleric acid, isobutyric acid or acetyl.

In certain embodiments, X is an amino acid sequence of Formula IV:

X1-Thr-His-X4-X5-X6-X7-X8-Phe-X10 (IV)

wherein
X1 is Asp, Ida, pGlu, bhAsp or absent;
X4 is Phe or Dpa;
X5 is Pro or bhPro;
X6 is Ile, Cys, or Arg;
X7 is Cys, Ile, Leu, or Val;
X8 is Ile, Lys, Glu, Phe, Gln, or Arg; and
X10 is Lys or absent.

In certain embodiments, X is an amino acid sequence of Formula V:

X1-Thr-His-X4-X5-Cys-Ile-X8-Phe-X10 (V)

wherein
X1 is Asp, Ida, pGlu, bhAsp, or absent;
X4 is Phe or Dpa;
X5 is Pro or bhPro;
X8 is Ile, Lys, Glu, Phe, Gln or Arg; and
X10 is Lys or absent.

In certain embodiments, the peptide comprises Formula VI:

R¹-X-Y-R² (VI)

or a pharmaceutically acceptable salt thereof,
wherein:
   R¹ is hydrogen, isovaleric acid, isobutyric acid, or acetyl;
   R² is NH₂ or OH;
   X is an amino acid sequence of Formula VII:

      X1-Thr-His-X4-X5-Cys-Ile-X8-Phe-X10 (VII)
   wherein
   X1 is Asp, Ida, pGlu, bhAsp, or absent:
      X4 is Phe or Dpa:
      X5 is Pro or bhPro;
      X8 is Ile, Lys, Glu, Phe, Gln, or Arg; and
      X10 is Lys or absent:
         wherein Y is an amino acid sequence of Formula VIII:

            Y1-Pro-Y3-Ser-Y5-Y6-Y7-Y8-Cys-Y10 (VIII)
         wherein
         Y1 is Gly, Glu, Val, or Lys;
         Y3 is Arg or Lys;
         Y5 is Arg or Lys;
         Y6 is Gly, Ser, Lys, Ile, or Arg;
         Y7 is Trp or absent;
         Y8 is Val, Thr, Asp, Glu, or absent; and
         Y10 is Lys or absent;
         wherein the peptide comprises a disulfide bond between the two Cys:
            wherein the peptide is optionally PEGylated on R¹, X, or Y:
            wherein a side chain of an amino acid of the peptide is optionally conjugated to a lipophilic substituent or polymeric moiety; and
            wherein Ida is iminodiacetic acid; pGlu is pyroglutamic acid: bhAsp is β-homoaspartic acid; and bhPro is β-homoproline.

In certain embodiments, the peptide comprises or consists of one of the following sequences:
DTHFPICIFGPRSKGWVC (SEQ ID NO: 46);
DTHFPCIIFGPRSKGWVCK (SEQ ID NO: 47);
DTHFPCIIFEPRSKGWVCK (SEQ ID NO: 48);
DTHFPCIIFGPRSKGWACK (SEQ ID NO: 49);
DTHFPCIIFGPRSKGWVCKK (SEQ ID NO:50);
DTHFPCIIFVCHRPKGCYRRVCR (SEQ ID NO: 51);
DTHFPCIKFGPRSKGWVCK (SEQ ID NO: 52);
DTHFPCIKFKPRSKGWVCK (SEQ ID NO: 53);
DTHFPCIIFGPRSRGWVCK (SEQ ID NO: 54);
DTHFPCIKFGPKSKGWVCK (SEQ ID NO: 55);
DTHFPCIKFEPRSKGCK (SEQ ID NO: 56);
DTHFPCIKFEPKSKGWECK (SEQ ID NO: 57);
DTHFPCIKFEPRSKKCK (SEQ ID NO: 58);
DTHFPCIKFEPRSKGCKK (SEQ ID NO: 59);
DTHFPCIKFKPRSKGCK (SEQ ID NO: 60);
DTHFPCIKFEPKSKGCK (SEQ ID NO: 61);
DTHFPCIKF (SEQ ID NO: 62);
DTHFPCIIF (SEQ ID NO: 63); or
DTKFPCIIF (SEQ ID NO: 64),
wherein said peptide is optionally PEGylated on R1, X, or Y:
   wherein a side chain of an amino acid of the peptide is optionally conjugated to a lipophilic substituent or polymeric moiety; and
   wherein the peptide optionally comprises a disulfide bond between two Cys amino acid residues of the peptide.

In certain embodiments, the peptide comprises or consists of one of the following sequences:
Isovaleric acid-DTHFPICIFGPRSKGWVC-NH₂ (SEQ ID NO:1);
Isovaleric acid-DTHFPCIIFGPRSKGWVCK-NH₂ (SEQ ID NO:2):
Isovaleric acid-DTHFPCIIFEPRSKGWVCK-NH₂ (SEQ ID NO:3);
Isovaleric acid-DTHFPCIIFGPRSKGWACK-NH₂ (SEQ ID NO:4);
Isovaleric acid-DTHFPCIIFGPRSKGWVCKK-NH₂ (SEQ ID NO:5);
Isovaleric acid-DTHFPCIIFVCHRPKGCYRRVCR-NH₂ (SEQ ID NO:6);
Isovaleric acid-DTHFPCI(K(PEG8))FGPRSKGWVCK-NH₂ (SEQ ID NO:7);
Isovaleric acid-DTHFPCIKF(K(PEG8))PRSKGWVCK-NH₂ (SEQ ID NO:8);
Isovaleric acid-DTHFPICIFGPRS(K(PEG8))GWVC-NH₂ (SEQ ID NO:9);
Isovaleric acid-DTHFPICIFGPRS(K(PEG4))GWVC-NH₂ (SEQ ID NO: 10);
Isovaleric acid-DTHFPCIIFGPRSRGWVC(K(PEG8))-NH₂ (SEQ ID NO:11);
Isovaleric acid-DTHFPCIIFGPRSRGWVC(K(PEG4))-NH₂ (SEQ ID NO:12);
Isovaleric acid-DTHFPCIIFGPRSRGWVC(K(PEG2))-NH₂ (SEQ ID NO:13);
Isovaleric acid-DTHFPCI(K(Palm))FGPRSKGWVCK-NH₂ (SEQ ID NO: 14),
Isovaleric acid-DTHFPCIKF)K(Palm))PRSKGWVCK-NH₂ (SEQ ID NO:15);
Isovaleric acid-DTHFPCIKFGP(K(Palm))SKGWVCK-NH₂ (SEQ ID NO:16);
Isovaleric acid-DTHFPCIKFGPRS(K(Palm))GWVCK-NH₂ (SEQ ID NO:17);
Isovaleric acid-DTHFPCIKFGPRSKGWVC(K(Palm))NH₂ (SEQ ID NO:18);
Isovaleric acid-DTHFPCI(K(PEG3-Palm))FGPRSKGWVCK-NH₂ (SEQ ID NO:19)
Isovaleric acid-DTHFPCIKF(K(PEG3-Palm))PRSKGWVCK-NH₂ (SEQ ID NO:20);
Isovaleric acid-DTHFPCIKFGP(K(PEG3-Palm))SKGWVCK-NH₂ (SEQ ID NO:21);
Isovaleric acid-DTHFPCIKFGPRS(K(PEG3-Palm))GWVCK-NH₂ (SEQ ID NO:22);
Isovaleric acid-DTHFPCIKFGPRSKGWVC(K(PEG3-Palm))-NH₂ (SEQ ID NO:23);
Isovaleric acid-DTHFPCIKFGPRSKGWVC(K(PEG8))-NH₂ (SEQ ID NO:24);
Isovaleric acid-DTHFPCI(K(isoGlu-Palm))FEPRSKGCK-NH₂ (SEQ ID NO:25):
Isovaleric acid-DTHFPCIKF-K(isoGlu-Palm)-PRSKGCK-NH₂ (SEQ ID NO:26):
Isovaleric acid-DTHFPCIKFEP(K(isoGlu-Palm))SKGCK-NH₂ (SEQ ID NO:27):
Isovaleric acid-DTHFPCIKFEP(K(isoGlu-Palm))SKGWECK-NH₂ (SEQ ID NO:28):
Isovaleric acid-DTHFPCIKFEPRS(K(isoGlu-Palm))GCK-NH₂ (SEQ ID NO:29):
Isovaleric acid-DTHFPCIKFEPRSK(K(isoGlu-Palm))CK-NH₂ (SEQ ID NO:30);
Isovaleric acid-DTHFPCIKFEPRSKGCK(K(isoGlu-Palm))-NH₂ (SEQ ID NO:31);
Isovaleric acid-DTHFPCI-K(Dapa-Palm)-FEPRSKGCK-NH₂ (SEQ ID NO:32);
Isovaleric acid-DTHFPCIK(F(Dapa-Palm))PRSKGCK-NH₂ (SEQ ID NO:33);
Isovaleric acid-DTHFPCIKFEP(K(Dapa-Palm))SKGCK-NH₂ (SEQ ID NO:34);
Isovaleric acid-DTHFPCIKFEPRS(K(Dapa-Palm))GCK-NH₂ (SEQ ID NO:35);
Isovaleric acid-DTHFPCIKFEPRSK(K(Dapa-Palm))CK-NH₂ (SEQ ID NO:36);
Isovaleric acid-DTHFPCIKFEPRSKGC(K(Dapa-Palm))K-NH₂ (SEQ ID NO:37);
Isovaleric acid-DTHFPCIKFEPRSKGC(K(Dapa-Palm))-NH₂ (SEQ ID NO:38);
Isovaleric acid-DTHFPCIKF(K(PEG11-Palm))PRSK[Sar]CK-NH₂ (SEQ ID NO:39);
Isolvaleric acid-DTHFPCIKF-NH₂ (SEQ ID NO:40):
Hy-DTHFPCIKF-NH₂ (SEQ ID NO:41);
Isolvaleric acid-DTHFPCIIF-NH₂ (SEQ ID NO:42);
Hy-DTHFPCIIKF-NH₂ (SEQ ID NO:43);
Isovaleric acid-DTKFPCIIF-NH₂ (SEQ ID NO:44); or
Hy-DTKFPCIIF-NH₂ (SEQ ID NO:45),
optionally wherein the peptide comprises a disulfide bond between two Cys amino acid residues of the peptide.

In certain embodiments, the peptide is selected from the group consisting of:
(a) 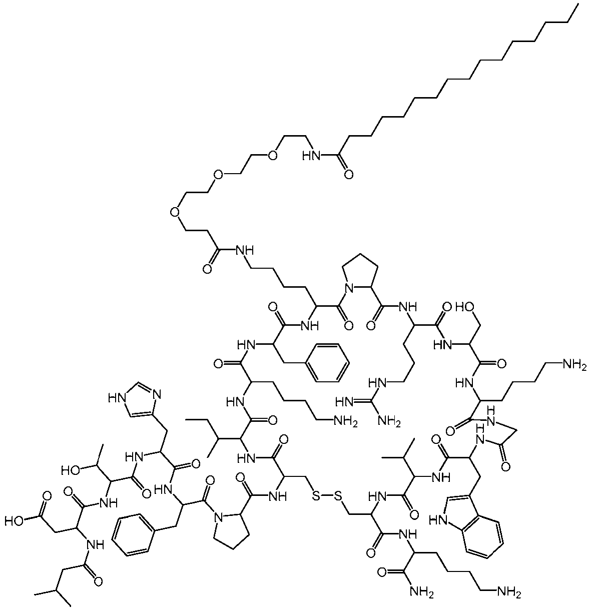 Isovaleric acid-DTHFPCIKF(K(PEG3-Palm))PRSKGWVCK-NH₂ (SEQ ID NO: 20);
(b) 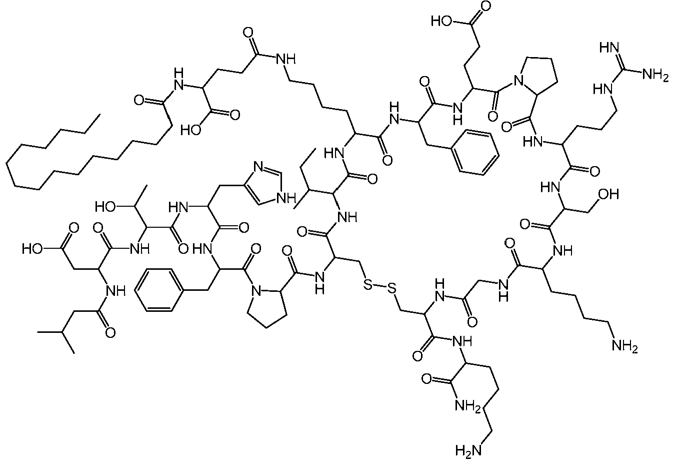 Isovaleric acid-DTHFPCI(K(isoGlu-Palm))FEPRSKGCK-NH₂ (SEQ ID NO: 25);
(c) 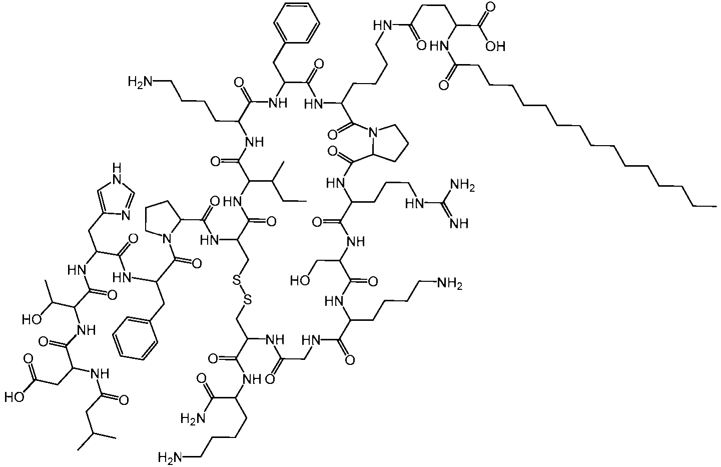 Isovaleric acid-DTHFPCIKF(K(isoGlu-Palm))PRSKGCK-NH₂ (SEQ ID NO: 26);
(d) 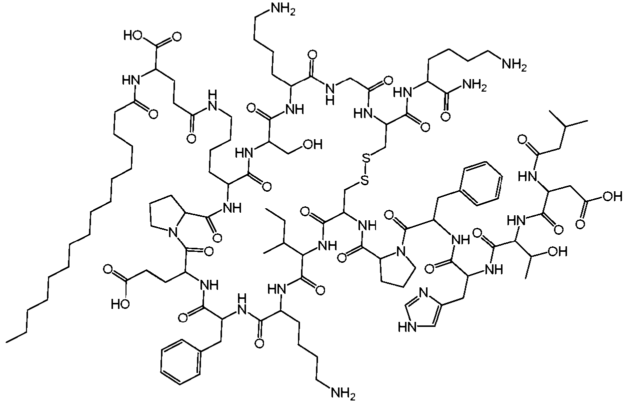 Isovaleric acid-DTHFPCIKFEP(K(isoGlu-Palm))SKGCK-NH₂ (SEQ ID NO: 27); and
(e) 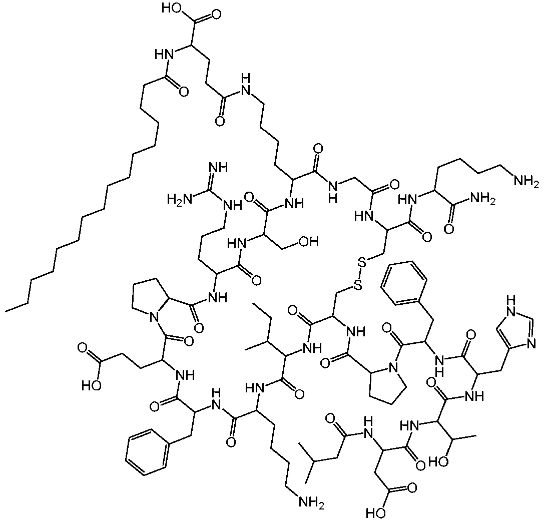 Isovaleric acid-DTHFPCIKFEPRS(K(isoGlu-Palm))GCK-NH₂ (SEQ ID NO: 29),
wherein the amino acids are L-amino acids.

Peptides disclosed herein, including hepcidin mimetics, may be produced using methods known in the art including chemical synthesis, biosynthesis or in vitro synthesis using recombinant DNA methods, and solid phase synthesis. See e.g., PCT Application Publication Nos. WO 2014/145561 and WO 2015/200916; Kelly & Winkler (1990) Genetic Engineering Principles and Methods, vol. 12, J. K. Setlow ed., Plenum Press, NY, pp. 1-19: Merrifield (1964) J Amer Chem Soc 85:2149; Houghten (1985) PNAS USA 82:5131-5135; and Stewart & Young (1984) Solid Phase Peptide Synthesis, 2ed. Pierce, Rockford, IL, which are herein incorporated by reference. The peptides disclosed herein may be purified using protein purification techniques known in the art such as reverse phase high-performance liquid chromatography (HPLC), ion-exchange or immunoaffinity chromatography, filtration or size exclusion, or electrophoresis. See Olsnes, S. and A. Pihl (1973) Biochem. 12(16):3121-3126; and Scopes (1982) Protein Purification, Springer- Verlag, NY, which are herein incorporated by reference. Alternatively, the peptides may be made by recombinant DNA techniques known in the art.

In certain embodiments, peptides disclosed herein may be PEGylated. As used herein, "Polyethylene glycol" or "PEG" is a polyether compound of general Formula H-(O-CH2-CH2)n-OH. PEGs are also known as polyethylene oxides (PEOs) or polyoxyethylenes (POEs), depending on their molecular weight. PEG, PEO, or POE, as used herein, refers to an oligomer or polymer of ethylene oxide. The three names are chemically synonymous, but PEG has tended to refer to oligomers and polymers with a molecular mass below 20,000 Da, PEO to polymers with a molecular mass above 20,000 Da, and POE to a polymer of any molecular mass. PEG and PEO are liquids or low-melting solids, depending on their molecular weights. Throughout this disclosure, the three names are used indistinguishably. PEGs are prepared by polymerization of ethylene oxide and are commercially available over a wide range of molecular weights from 300 Da to 10,000,000 Da. While PEG and PEO with different molecular weights find use in different applications, and have different physical properties (e.g., viscosity) due to chain length effects, their chemical properties are nearly identical. PEG moieties include polyethylene glycols (PEG), homo- or co-polymers of PEG, a monomethyl-substituted polymer of PEG (mPEG), or polyoxyethylene glycerol (POG). See, for example, Int. J. Hematology 68:1 (1998); Bioconjugate Chem. 6:150 (1995); and Crit. Rev. Therap. Drug Carrier Sys. 9:249 (1992). Also encompassed are PEGs that are prepared for purpose of half life extension, for example, mono-activated, alkoxy-terminated polyalkylene oxides (POAs) such as mono-methoxy-terminated polyethyelene glycols (mPEGs): bis activated polyethylene oxides (glycols) or other PEG derivatives are also contemplated. Suitable PEGs will vary substantially by weights, e.g., ranging from about 200 Da to about 40,000 Da or from about 200 Da to about 60,000 Da, any of which may used for the purposes of the present disclosure. In certain embodiments, PEGs having molecular weights from 200 Da to 2,000 Da or from 200 Da to 500 Da are used. Different forms of PEG may also be used, depending on the initiator used for the polymerization process: a common initiator is a monofunctional methyl ether PEG, or methoxypoly(ethylene glycol), abbreviated mPEG. Lower-molecular-weight PEGs are also available as pure oligomers, referred to as monodisperse, uniform, or discrete. These are used in certain embodiments of the present disclosure.

PEGs are also available with different geometries: branched PEGs have three to ten PEG chains emanating from a central core group: star PEGs have 10 to 100 PEG chains emanating from a central core group; and comb PEGs have multiple PEG chains normally grafted onto a polymer backbone. PEGs can also be linear. The numbers that are often included in the names of PEGs indicate their average molecular weights (e.g., a PEG with n = 9) would have an average molecular weight of approximately 400 daltons, and would be labeled PEG 400.

As used herein, "PEGylation" is the act of covalently coupling a PEG structure to the peptide inhibitor of the invention, which is then referred to as a "PEGylated peptide inhibitor". In certain embodiments, the PEG of the PEGylated side chain is a PEG with a molecular weight from about 200 Da to about 40,000 Da.

In various embodiments, the agents are present in a pharmaceutical composition comprising one or more pharmaceutically acceptable diluents, carriers, or excipients. A pharmaceutically acceptable carrier, diluent or excipient refers to a non-toxic solid, semi-solid or liquid filler, diluent, encapsulating material or formulation auxiliary of any type. The term "pharmaceutically acceptable carrier" includes any of the standard pharmaceutical carriers. Pharmaceutically acceptable carriers for therapeutic use are well known in the pharmaceutical art and are described, for example, in "Remington's Pharmaceutical Sciences", 17th edition, Alfonso R. Gennaro (Ed.), Mark Publishing Company, Easton, PA, USA, 1985. For example, sterile saline and phosphate-buffered saline at slightly acidic or physiological pH may be used. Suitable pH-buffering agents may, e.g., be phosphate, citrate, acetate, tris(hydroxymethyl)aminomethane (TRIS), N-tris(hydroxymethyl)methyl-3-aminopropanesulfonic acid (TAPS), ammonium bicarbonate, diethanolamine, histidine, arginine, lysine or acetate (e.g., as sodium acetate), or mixtures thereof. The term further encompasses any carrier agents listed in the US Pharmacopeia for use in animals, including humans.

### EXAMPLES

The following examples demonstrate certain specific embodiments of the present invention. The following examples were carried out using standard techniques that are well known and routine to those of skill in the art, except where otherwise described in detail. It is to be understood that these examples are for illustrative purposes only and do not purport to be wholly definitive as to conditions or scope of the invention. As such, they should not be construed in any way as limiting the scope of the present invention.

### ABBREVIATIONS:

- DCM:: dichloromethane
- DMF:: N,N-dimethylformamide
- NMP:: N-methylpyrolidone
- HBTU:: O-(Benzotriazol-1-yl)-N,N,N',N'-tetramethyluronium hexafluorophosphate
- HATU:: 2-(7-aza-1H-benzotriazole-1-yl)-1,1,3,3-tetramethyluronium hexafluorophosphate
- DCC:: Dicyclohexylcarbodiimide
- NHS:: N-hydoxysuccinimide
- DIPEA:: diisopropylethylamine
- EtOH:: ethanol
- Et2O:: diethyl ether
- Hy:: hydrogen
- TFA:: trifluoroacetic acid
- TIS:: triisopropylsilane
- ACN:: acetonitrile
- HPLC:: high performance liquid chromatography
- ESI-MS:: electron spray ionization mass spectrometry
- PBS:: phosphate-buffered saline
- Boc:: t-butoxycarbonyl
- Fmoc:: Fluorenylmethyloxycarbonyl
- Acm:: acetamidomethyl
- IVA:: Isovaleric acid (or Isovaleryl)

K( ): In the peptide sequences provided herein, wherein a compound or chemical group is presented in parentheses directly after a Lysine residue, it is to be understood that the compound or chemical group in the parentheses is a side chain conjugated to the Lysine residue. So, e.g., but not to be limited in any way, K-[(PEG8)]- indicates that a PEG8 moiety is conjugated to a side chain of this Lysine.

Palm: Indicates conjugation of a palmitic acid (palmitoyl).

As used herein "C( )" refers to a cysteine residue involved in a particular disulfide bridge. For example, in Hepcidin, there are four disulfide bridges: the first between the two C(1) residues; the second between the two C(2) residues: the third between the two C(3) residues; and the fourth between the two C(4) residues. Accordingly, in some embodiments, the sequence for Hepcidin is written as follows:
Hy-DTHFPIC(1)IFC(2)C(3)GC(2)C(4)HRSKC(3)GMC(4)C(1)KT-OH (SEQ ID NO: 65); and the sequence for other peptides may also optionally be written in the same manner.

### EXAMPLE 1

### SYNTHESIS OF PEPTIDE ANALOGUES

Unless otherwise specified, reagents and solvents employed in the following were available commercially in standard laboratory reagent or analytical grade, and were used without further purification.

### Procedure for solid-phase synthesis of peptides

Peptide analogues of the invention were chemically synthesized using optimized 9-fluorenylmethoxy carbonyl (Fmoc) solid phase peptide synthesis protocols. For C-terminal amides, rink-amide resin was used, although wang and trityl resins were also used to produce C-terminal acids. The side chain protecting groups were as follows: Glu, Thr and Tyr: O-tButyl: Trp and Lys: t-Boc (t-butyloxycarbonyl); Arg: N-gamma-2,2,4,6,7-pentamethyldihydrobenzofuran-5-sulfonyl; His, Gln, Asn, Cys: Trityl. For selective disulfide bridge formation, Acm (acetamidomethyl) was also used as a Cys protecting group. For coupling, a four to ten-fold excess of a solution containing Fmoc amino acid, HBTU and DIPEA (1:1:1.1) in DMF was added to swelled resin [HBTU: O-(Benzotriazol-1-yl)-N,N,N',N'-tetramethyluronium hexafluorophosphate; DIPEA: diisopropylethylamine: DMF: dimethylformamide]. HATU (O-(7-azabenzotriazol-1-yl)-1,1,3,3,-tetramethyluronium hexafluorophosphate) was used instead of HBTU to improve coupling efficiency in difficult regions. Fmoc protecting group removal was achieved by treatment with a DMF, piperidine (2:1) solution.

### Procedure for cleavage of peptides off resin

Side chain deprotection and cleavage of the peptide analogues of the invention (e.g., Compound No. 2) was achieved by stirring dry resin in a solution containing trifluoroacetic acid, water, ethanedithiol and tri-isopropylsilane (90:5:2.5:2.5) for 2 to 4 hours. Following TFA removal, peptide was precipitated using ice-cold diethyl ether. The solution was centrifuged and the ether was decanted, followed by a second diethyl ether wash. The peptide was dissolved in an acetonitrile, water solution (1:1) containing 0.1% TFA (trifluoroacetic acid) and the resulting solution was filtered. The linear peptide quality was assessed using electrospray ionization mass spectrometry (ESI-MS).

### Procedure for purification of peptides

Purification of the peptides of the invention (e.g., Compound No. 2) was achieved using reverse-phase high performance liquid chromatography (RP-HPLC). Analysis was performed using a C18 column (3µm, 50 x 2mm) with a flow rate of 1 mL/min. Purification of the linear peptides was achieved using preparative RP-HPLC with a C18 column (5µm, 250 x 21.2 mm) with a flow rate of 20 mL/min. Separation was achieved using linear gradients of buffer B in A (Buffer A: Aqueous 0.05% TFA, Buffer B: 0.043% TFA, 90% acetonitrile in water).

### Procedure for oxidation of peptides.

Method A (Single disulfide oxidation). Oxidation of the unprotected peptides of the invention was achieved by adding drop-wise iodine in MeOH (1 mg per 1 mL) to the peptide in a solution (ACN: H₂O, 7: 3, 0.5% TFA). After stirring for 2 min, ascorbic acid portion wise was added until the solution was clear and the sample was immediately loaded onto the HPLC for purification.

Method B (Selective oxidation of two disulfides). When more than one disulfide was present, selective oxidation was often performed. Oxidation of the free cysteines was achieved at pH 7.6 NH₄CO₃ solution at 1mg /10 mL of peptide. After 24 h stirring and prior to purification the solution was acidified to pH 3 with TFA followed by lyophilization. The resulting single oxidized peptides (with ACM protected cysteines) were then oxidized / selective deprotection using iodine solution. The peptide (1 mg per 2 mL) was dissolved in MeOH/H₂0, 80:20 iodine dissolved in the reaction solvent was added to the reaction (final concentration: 5 mg/mL) at room temperature. The solution was stirred for 7 minutes before ascorbic acid was added portion wise until the solution is clear. The solution was then loaded directly onto the HPLC.

Method C (Native oxidation). When more than one disulfide was present and when not performing selective oxidations, native oxidation was performed. Native oxidation was achieved with 100 mM NH4CO3 (pH7.4) solution in the presence of oxidized and reduced glutathione (peptide/GSH/GSSG, 1:100:10 molar ratio) of (peptide: GSSG: GSH, 1:10, 100). After 24 h stirring and prior to RP-HPLC purification the solution was acidified to pH 3 with TFA followed by lyophilization.

Procedure of cysteine oxidation to produce dimers. Oxidation of the unprotected peptides of the invention was achieved by adding drop-wise iodine in MeOH (1 mg per 1 mL) to the peptide in a solution (ACN: H2O, 7: 3, 0.5% TFA). After stirring for 2 min, ascorbic acid portion wise was added until the solution was clear and the sample was immediately loaded onto the HPLC for purification.

### Procedure for dimerization.

Glyoxylic acid (DIG), IDA, or Fmoc-β-Ala-IDA was pre-activated as the N-hydoxysuccinimide ester by treating 1 equivalent (abbreviated "eq") of the acid with 2.2 eq of both N-hydoxysuccinimide (NHS) and dicyclohexyl carbodiimide (DCC) in NMP (N-methyl pyrolidone) at a 0.1 M final concentration. For the PEG13 and PEG25 linkers, these chemical entities were purchased pre-formed as the activated succinimide ester. The activated ester ~ 0.4 eq was added slowly to the peptide in NMP (1mg/mL) portionwise. The solution was left stirring for 10 min before 2-3 additional aliquots of the linker ~0.05 eq were slowly added. The solution was left stirring for a further 3 h before the solvent was removed under *vaccuo* and the residue was purified by reverse phase HPLC. An additional step of stirring the peptide in 20% piperidine in DMF (2 x 10 min) before an additional reverse phase HPLC purification was performed.

One of skill in the art will appreciate that standard methods of peptide synthesis may be used to generate the compounds of the invention.

### Linker activation and dimerization

Peptide monomer subunits were linked to form hepcidin analogue peptide dimers as described below.

Small Scale DIG Linker Activation Procedure: 5mL of NMP was added to a glass vial containing IDA diacid (304.2 mg, 1 mmol), N-hydroxysuccinimide (NHS, 253.2 mg, 2.2 eq. 2.2mmol) and a stirring bar. The mixture was stirred at room temperature to completely dissolve the solid starting materials. N, N'-Dicyclohexylcarbodiimide (DCC, 453.9mg, 2.2 eq., 2.2 mmol) was then added to the mixture. Precipitation appeared within 10 min and the reaction mixture was further stirred at room temperature overnight. The reaction mixture was then filtered to remove the precipitated dicyclohexylurea (DCU). The activated linker was kept in a closed vial prior to use for dimerization. The nominal concentration of the activated linker was approximately 0.20 M.

For dimerization using PEG linkers, there was no pre-activation step involved. Commercially available pre-activated bi-functional PEG linkers were used.

Dimerization Procedure: 2mL of anhydrous DMF was added to a vial containing peptide monomer (0.1 mmol). The pH of the peptide was the adjusted to 8~9 with DIEA. Activated linker (IDA or PEG13, PEG 25) (0.48eq relative to monomer, 0.048 mmol) was then added to the monomer solution. The reaction mixture was stirred at room temperature for one hour. Completion of the dimerization reaction was monitored using analytical HPLC. The time for completion of dimerization reaction varied depending upon the linker. After completion of reaction, the peptide was precipitated in cold ether and centrifuged. The supernatant ether layer was discarded. The precipitation step was repeated twice. The crude dimer was then purified using reverse phase HPLC (Luna C18 support, 10u, 100A, Mobile phase A: water containing 0.1% TFA, mobile phase B: Acetonitrile (ACN) containing 0.1% TFA, gradient of 15%B and change to 45%B over 60min, flow rate 15ml/min). Fractions containing pure product were then freeze-dried on a lyophilizer.

### Conjugation of Half-Life Extension Moieties

Conjugation of peptides were performed on resin. Lys(ivDde) was used as the key amino acid. After assembly of the peptide on resin, selective deprotection of the ivDde group occurred using 3 x 5 min 2% hydrazine in DMF for 5 min. Activation and acylation of the linker using HBTU, DIEA 1-2 equivalents for 3 h, and Fmoc removal followed by a second acylation with the lipidic acid gave the conjugated peptide.

### EXAMPLE 2

### PHARMACODYNAMICS OF A HEPCIDIN MIMETIC PEPTIDE IN HEREDITARY HEMOCHROMATOSIS MICE

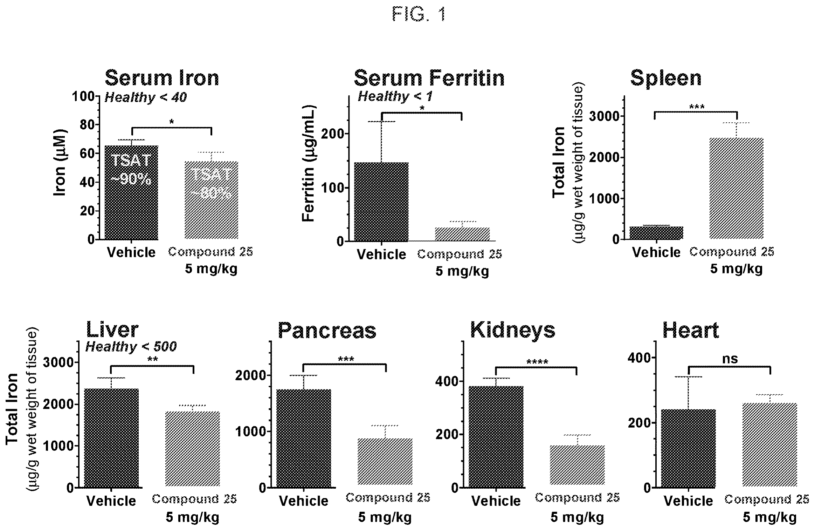

The ability of hepcidin mimetics to modulate iron and other markers was determined in a mouse model of hereditary hemochromatosis (HH). HH mice (129S-Hjv^{tm1Nca}/J) were maintained under low iron diet (~2ppm iron) until 8 weeks of age, then iron-loaded for 5 weeks with normal diet (~260ppm iron). Mice were then treated with Compound 25 (2.5 mg/kg, Q2D) under normal iron diet. All measurements were at 48 hr post-last dose (at trough drug levels). Compound 25 caused sustained reductions in TSAT% and redistribution of iron to spleen under iron-overload conditions in hereditary hemochromatosis (HH) mice (FIG. 1). In iron-overloaded HH mice, TSAT% was reduced by ~10% at trough drug levels, after 12 doses of Compound 25 (5 mg/kg; Q2D). This was accompanied by lowered ferritin, and redistribution of iron to spleen. Iron accumulation was reduced in liver, pancreas, and kidneys.

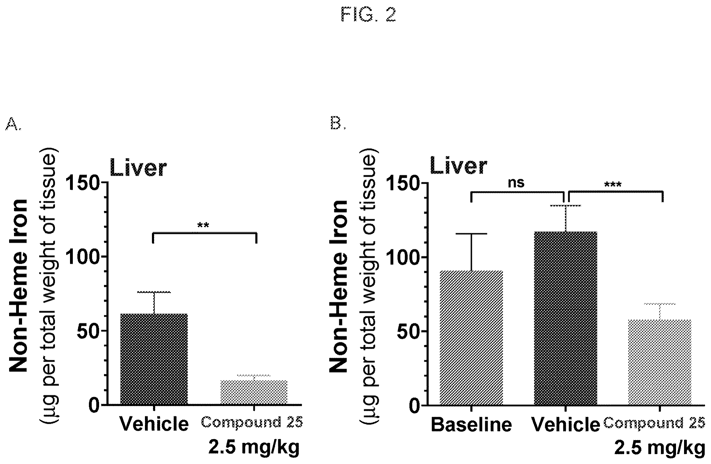

In HH mice, under less severe iron-overload conditions, liver iron accumulation was prevented in groups treated with Compound 25 for 2 weeks (FIGs. 2A and 2B). HH mice were iron loaded to different levels by maintaining them under either low iron or normal iron diets for two weeks to achieve different levels of iron overload at the start point of therapy. Mice were then treated with Compound 25 (2.5 mg/kg, Q2D) under normal iron diet. Organ iron concentrations were assessed by ICP-MS method (total iron) or colorimetric assays (non-heme iron). Statistical analysis: Oneway ANOVA w/Dunnett's Multiple Comparisons or t test w/ Welch's correction.

As shown in FIG. 2A, "non-diseased" mice that were maintained on a low-iron diet to prevent iron accumulation until start of therapy showed a reduction in liver iron, demonstrating that Compound 25 prevents hyperabsorption of dietary iron and therefore prevents elevated TSAT% and iron accumulation in liver. As shown in FIG. 2B, mice maintained on normal diet for two weeks before treatment, to allow for partial iron overload, also showed a reduction in iron liver. In this case, Compound 25 not only prevented further iron accumulation in liver (as compared to vehicle) but also redistributed the iron away from liver.

These studies demonstrate that starting from a high iron overloaded condition (TSAT% > 90%; severe disease), lowered TSAT%, which is a consequence of locking iron in the splenic macrophages (that are equipped to store high amount of iron) is associated with a reduction of iron accumulation in organs (e.g., pancreas, kidneys.) They also show that TSAT% at trough drug levels, particularly below normal levels, is a serum biomarker of clinical efficacy (e.g., organ iron overload).

These studies show that treatment with a hepcidin mimetic peptide, e.g., Compound 25, will potentially benefit hemochromatosis patients with primary and secondary iron overload, e.g., by lowering serum TSAT% and labile iron through sequestration of iron in splenic macrophages, to prevent and reverse iron loading.

### EXAMPLE 3

### EFFICACY OF A HEPCIDIN MIMETIC PEPTIDE IN HEREDITARY HEMOCHROMATOSIS PATIENTS

A clinical efficacy study was conducted in HH patients. Subjects received subcutaneous Compound 25 for up to 24 weeks. Subjects were started at an initial dose of 10 mg per week subcutaneously. The dose was increased to 20 mg per week, and subsequently to 40 mg and to 80 mg per week as needed, based on tolerability and the pharmacodynamic marker TSAT. In addition, subcutaneous dosing schedules of 10 mg, 20 mg, 30, and 40 mg twice weekly (Day 1 and either Day 4 or Day 5 were tested). The majority of patients were administered doses of 20 mg per week or less. Subjects' safety and blood iron parameters (serum iron, serum ferritin, transferrin, and TSAT) were collected to monitor the pharmacodynamic effect of Compound 25. Effects on phlebotomy need and QoL data (36-Item Short Form Health Survey [SF-36], and Patient's Global Impression of Change [PGI-C]) were also be collected and tabulated.

Individual subject's dose and schedule were determined based on the pharmacodynamic (PD) marker TSAT measured at two time points following dosing: once at peak PD effect one day post dose and once at trough PD effect. The intent of dose and schedule adjustment was to reduce TSAT and serum iron levels. If necessary, the dose of Compound 25 was increased weekly in a sequential manner from 10 mg to 20 mg and, if necessary, to 30 mg, 40 mg and 80 mg until the TSAT was less than approximately 40% at peak PD effect one day post dose and at the trough PD effect prior to the next dose of Compound 25. Dosing was once a week or twice a week. The trough PD effect was the TSAT value measured 7 days after the Compound 25 dose (and before the next dose) for once weekly dosing or 7 days after the first dose in a week for twice weekly dosing. For the twice weekly regimen, the doses were given at least 3 days apart (e.g., on Day 1 and Day 4 or 5 of each week). The twice weekly Compound 25 dose was escalated to a maximum of 40 mg twice weekly. To facilitate dose escalation and the identification of a therapeutic dose, investigators assessed TSAT values on days subjects came to the clinic for any dose adjustments.

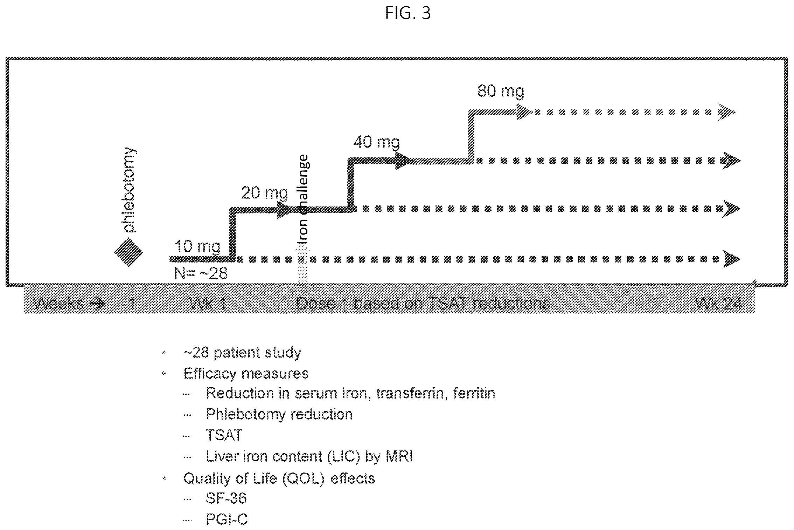

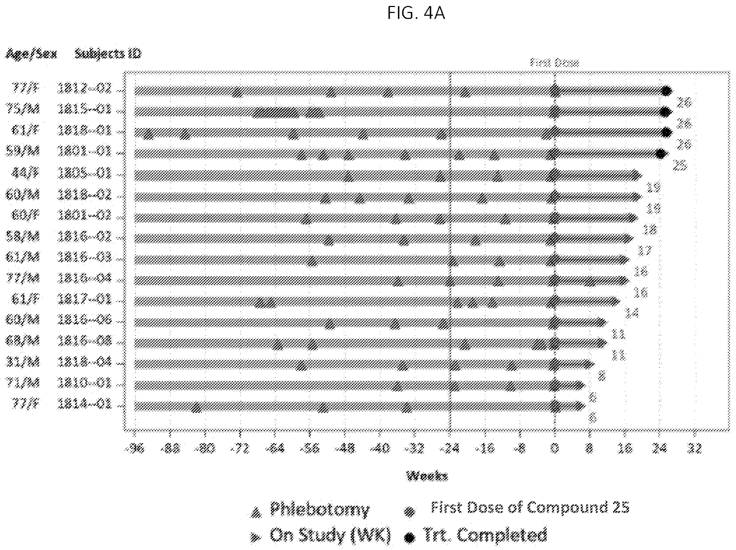

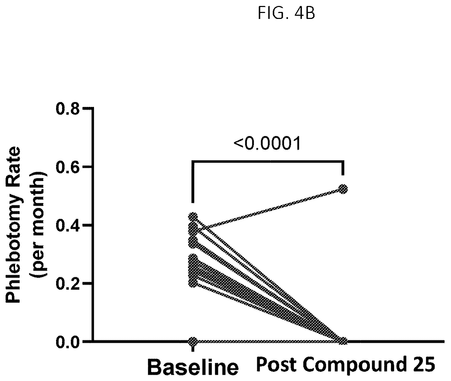

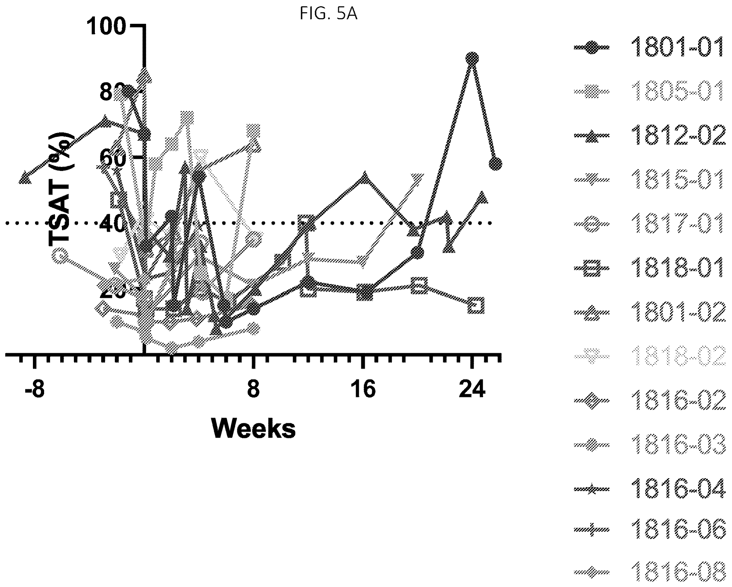

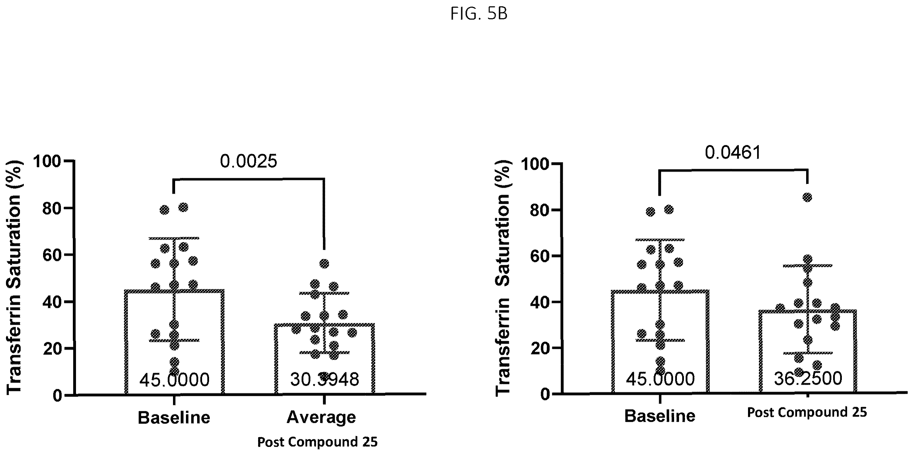

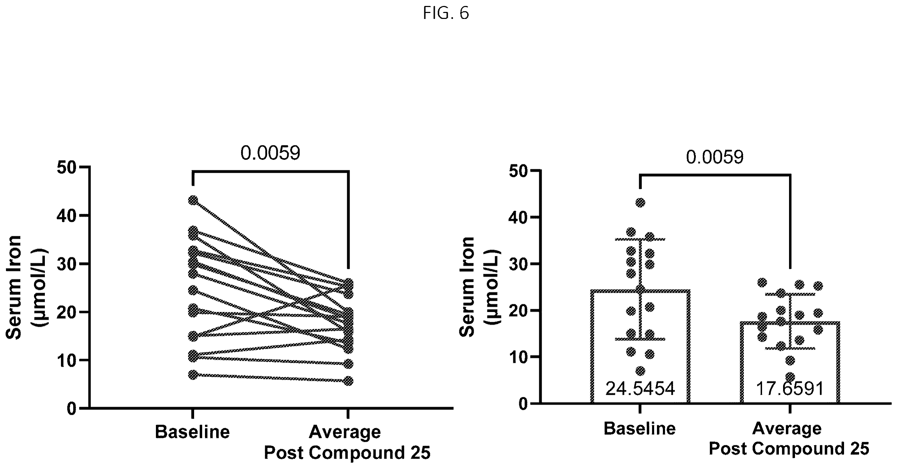

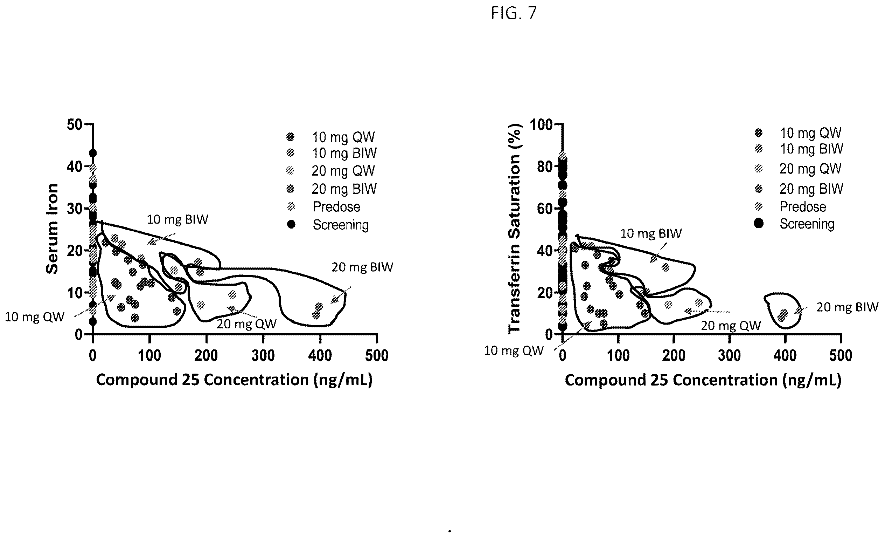

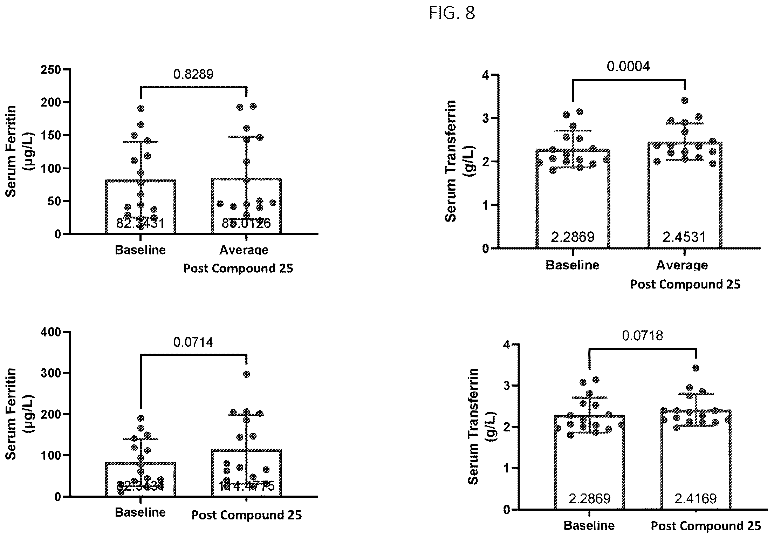

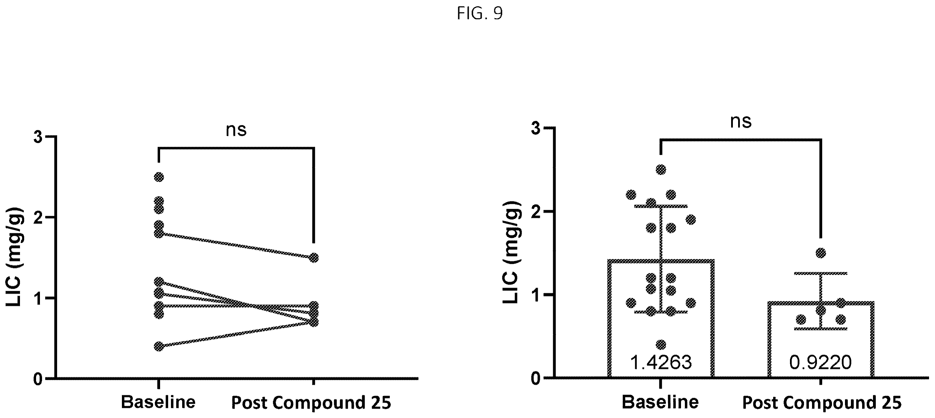

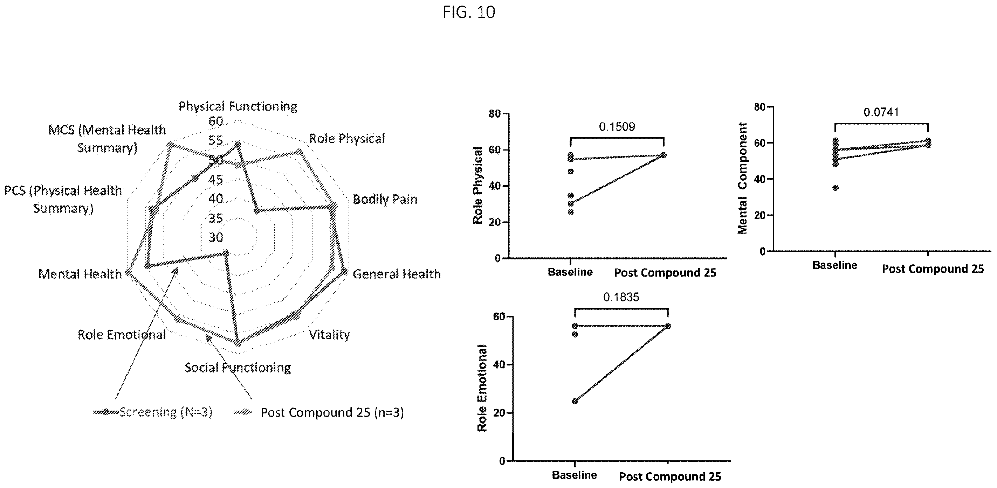

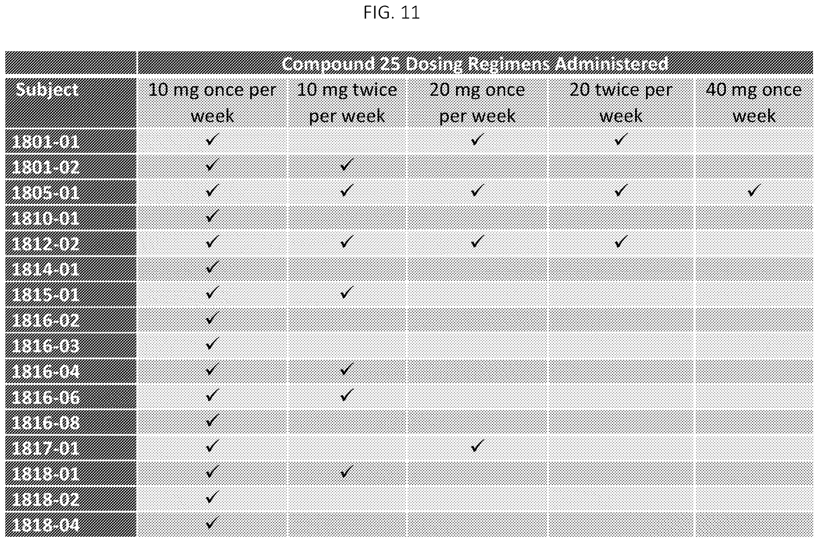

Patients had a prior phlebotomy frequency of at least 0.25 per month (e.g., received at least three phlebotomies over the previous 12 months, or at least four phlebotomies over the previous 15 months) and a phlebotomy frequency of less than 1 per month, a hemoglobin >11.5 g/dL, and serum ferritin <300 ng/mL at screening (before screening phlebotomy).
Methods: This single-arm, open-label, dose-finding phase 2 study investigated subcutaneous hepcidin mimetic (Compound 25) in patients (pts) with confirmed HH who were on documented stable phlebotomies (phl) for at least 6 months (mos) prior to treatment with a phl frequency of 0.25 - 1 per month. Patients with clinically meaningful laboratory abnormalities and those receiving iron chelation therapy or erythrocytapheresis were excluded. Patients received individually titrated Compound 25 doses once or twice a week to maintain transferrin saturation (TSAT) below 45% and were followed for 6 months, as outlined in FIG. 3. Individual doses are shown in FIG. 11. Endpoints included TSAT, serum iron, serum transferrin and serum ferritin, liver iron content (LIC) measured by FerriScan MRI, adverse events, and patient-reported outcomes on the Patient Global Impression of change (PGI-C) and Medical Outcome Study Questionnaire Short Form Health Survey (SF-36). Compound 25 was formulated in an aqueous buffered solution.
Results: Sixteen pts (10 M/6 F) were enrolled. Mean age and weight were 62.5 years and 88.1 kg, respectively. LIC values were maintained at pre-study levels, with minimal use of phlebotomies during the duration of the study. In the 6 months prior to the study, average phlebotomy (phl) rate was 0.27 phl/month prior to study compared to 0.03 phb/month during study (p<0.0001). Study endpoints included safety, reduction in phlebotomies, serum iron, TSAT, transferrin, ferritin, liver iron content by MRI, adverse events. Compound 25 was able to eliminate phlebotomy in the vast majority of subjects for the duration of treatment (FIGs. 4A and 4B). Treatment with Compound 25 also showed a statistically significant reduction in mean TSAT level (FIGs. 5A and 5B). Average baseline TSAT was 45% compared with 30.4% during the study (p=0.0025). For certain pts with TSAT above 45% at baseline, treatment with Compound 25 reduced TSAT to less than or close to the 45% when phlebotomies could not. Treatment with Compound 25 further resulted in a reduction in mean serum iron (FIG. 6). Serum iron was reduced from 24.5 µmol/L pre-study to an average of 17.7 µmol/mL during the study (p=0.0059), or from 137 ug/dL at baseline to 98.6 ug/dL post treatment with Compound 25. There was a dose- and concentration-dependent decrease in serum iron and TSAT (FIG. 7). Serum ferritin and serum transferrin levels maintained relatively constant from baseline to post-treatment with Compound 25 (FIG. 8). Treatment with Compound 25 maintained liver iron content with no statistically significant difference at baseline or post treatment with Compound 25 (FIG. 9). There were no notable changes in hematological parameters such as hematocrit, erythrocytes, leucocytes, or platelets. Patient reported outcomes were determined, and improvements were noted in the SF-36 Role Physical and Role Emotional subcomponents following treatment with Compound 25 (FIG. 10). Compound 25 was generally well tolerated. All treatment-related adverse events were characterized as CTCAE grade 1 or 2.

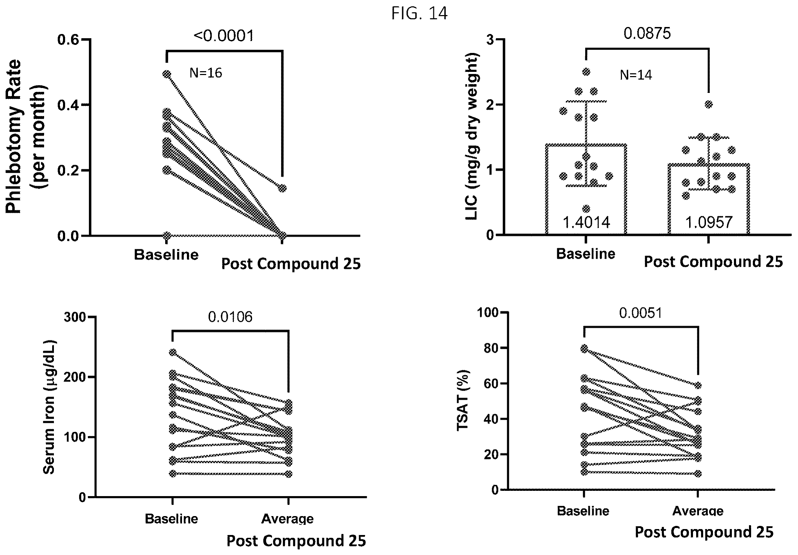

A summary of the results obtained from the six-month, open-label study in 16 HH patients in maintenance phase of iron depletion with stable pre-study phlebotomy for ≥6 months; requiring ≥3 phlebotomies/12 months or ≥4 phlebotomies/15 months is shown in FIG. 14.
Conclusion: Compound 25 demonstrated a pharmacologic effect in reducing serum iron and TSAT levels. These pharmacodynamic effects corresponded to a reduced need for phl, control in LIC, and in clinically meaningful changes in patient reported outcomes. These data indicate Compound 25 controls LIC in the absence of phl. Compound 25 was well tolerated in patients with HH. These data support Compound 25 and other hepcidin mimetics as a treatment for HH.

### EXAMPLE 4

### PHARMACODYNAMIC CONTROL OF A HEPCIDIN MIMETIC PEPTIDE

Iron is a significant component of normal cellular function and dysregulated metabolism contributes to disease formation and/or progression. Compound 25 controls iron stores in the body by blocking adsorption of dietary iron and rapid redistribution of serum iron into splenic macrophages. Restoration of iron homeostasis by Compound 25 has been demonstrated in subjects with healthy iron stores, iron deficiency, and tissue iron overload.

In this Example, the pharmacodynamic control of subcutaneously administered Compound was examined in healthy volunteers and HH patients.

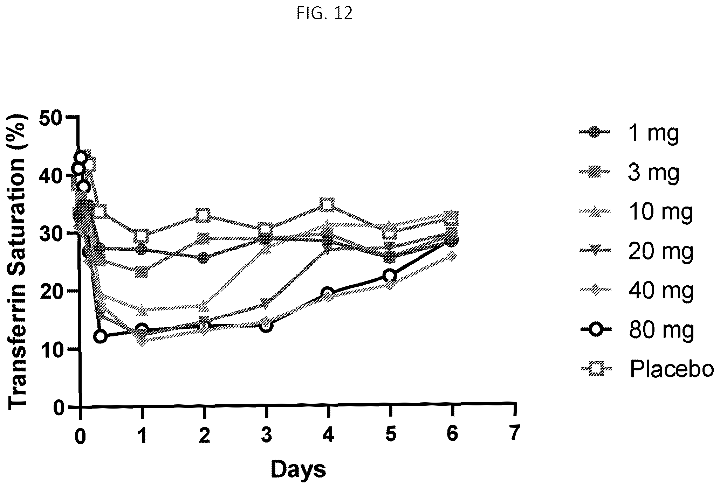

In a first study, healthy human volunteers were subcutaneously administered placebo or a single dose (1 mg, 3 mg, 10 mg, 20 mg, 40 mg, or 80 mg) of Compound 25. The subjects' serum TSAT levels were determined as a surrogate for serum iron stores over the six days following administration, and the subjects' mean corpuscular hemoglobin concentration (MCHC) levels were determined as a surrogate for red blood cell integrity over the six days following administration. As shown in FIG. 12, the subjects exhibited a dose responsive reduction in TSAT levels within less than one day, which gradually increased over the next 5-6 days.

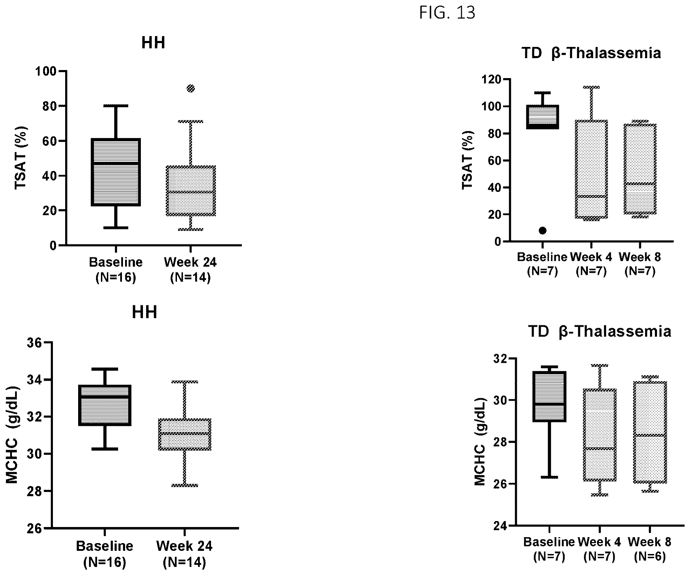

In a second study, HH patients in maintenance phase and transfusion-dependent β-thalassemia patients were subcutaneously administered Compound 25, and their TSAT and MCHC levels were determined prior to and 24 weeks after administration of Compound 25. As shown in FIG. 13, the subjects' TSAT levels and MCHC levels decreased following treatment.

These studies demonstrate dose-related and consistent pharmacodynamic control of Compound 25 in humans, and improvement in both serum iron stores and RBC integrity following treatment of HH patients with Compound 25, and support using Compound 25 and other hepcidin mimetics to treat HH, including but not limited to HH with excess iron and joint arthropathy.

All of the above U.S. patents, U.S. patent application publications, U.S. patent applications, foreign patents, foreign patent applications and non-patent publications referred to in this specification and/or listed in the Application Data Sheet, are incorporated herein by reference, in their entirety.

From the foregoing it will be appreciated that, although specific embodiments of the invention have been described herein for purposes of illustration, various modifications may be made without deviating from the spirit and scope of the invention.

The invention provides the following numbered embodiments:
1. A method for treating hereditary hemochromatosis, hereditary hemochromatosis arthropathy, or joint pain associated with hereditary hemochromatosis arthropathy, in a human subject, comprising administering to the subject an effective amount of a hepcidin mimetic, wherein the effective amount comprises a dose in the range of about 5 mg to about 40 mg, and optionally wherein the subject is administered different doses during different time periods over a course of treatment.
2. The method of embodiment 1, wherein the subject is administered the effective amount of the hepcidin mimetic about once a week or about twice a week for at least some time period during the course of treatment.
3. The method of embodiment 2, wherein the subject is administered about 5 mg to about 20 mg of the hepcidin mimetic about twice a week for at least some time period during the course of treatment.
4. The method of embodiment 2, wherein the subject is administered about 10 mg to about 40 mg of the hepcidin mimetic about once a week for at least some time period during the course of treatment.
5. The method of any one of embodiments 1-4, wherein the effective amount causes a decrease in the subject's transferrin saturation (TSAT) level and/or serum iron level.
6. The method of embodiment 5, wherein the subject's TSAT level is decreased to less than 45%.
7. The method of embodiment 5, wherein the subject's TSAT level is decreased to less than 40%.
8. The method of embodiment 6 or embodiment 7, wherein the subject's TSAT level is maintained at less than 45% over the course of treatment with the hepcidin mimetic, optionally wherein the course of treatment comprises at least 24 weeks.
9. The method of any one of embodiments 1-8, wherein hepcidin mimetic is a peptide having Formula I:

   R1-X-Y-R2 (I)

   or a pharmaceutically acceptable salt or solvate thereof,
   wherein
   R1 is hydrogen, a C1-C6 alkyl, a C6-C12 aryl, a C1-C20 alkanoyl, or pGlu;
   R2 is NH₂ or OH;
   X is an amino acid sequence of Formula II:

      X1-X2-X3-X4-X5-X6-X7-X8-X9-X10 (II)
   wherein
   X1 is Asp, Ala, Ida, pGlu, bhAsp, Leu, D-Asp, or absent;
   X2 is Thr, Ala, or D-Thr;
   X3 is His, Lys, D-His, or Lys;
   X4 is Phe, Ala, Dpa, or D-Phe;
   X5 is Pro, Gly, Arg, Lys, Ala, D-Pro, or bhPro;
   X6 is Ile, Cys, Arg, Lys, D-Ile, or D-Cys;
   X7 is Cys, Ile, Leu, Val, Phe, D-Ile, or D-Cys;
   X8 is Ile, Arg, Phe, Gln, Lys, Glu, Val, Leu, or D-Ile;
   X9 is Phe or bhPhe; and
   X10 is Lys, Phe, or absent;
   wherein if Y is absent, X7 is Ile; and
   Y is an amino acid sequence of Formula III:

      Y1-Y2-Y3-Y4-Y5-Y6-Y7-Y8-Y9-Y10-Y11-Y12-Y13-Y14-Y15 (III)
   wherein
   Y1 is Gly, Cys, Ala, Phe, Pro, Glu, Lys, D-Pro, Val, Ser, or absent;
   Y2 is Pro, Ala, Cys, Gly, or absent;
   Y3 is Arg, Lys, Pro, Gly, His, Ala, Trp, or absent;
   Y4 is Ser, Arg, Gly, Trp, Ala, His, Tyr, or absent;
   Y5 is Lys, Met, Arg, Ala, or absent;
   Y6 is Gly, Ser, Lys, Ile, Ala, Pro, Val, or absent;
   Y7 is Trp, Lys, Gly, Ala, Ile, Val, or absent;
   Y8 is Val, Thr, Gly, Cys, Met, Tyr, Ala, Glu, Lys, Asp, Arg, or absent;
   Y9 is Cys, Tyr, or absent;
   Y10 is Met, Lys, Arg, Tyr, or absent;
   Y11 is Arg, Met, Cys, Lys, or absent;
   Y12 is Arg, Lys, Ala or absent;
   Y13 is Arg, Cys, Lys, Val or absent;
   Y14 is Arg, Lys, Pro, Cys, Thr or absent; and
   Y15 is Thr, Arg or absent;
   wherein the peptide of Formula I is optionally PEGylated on R1, X, or Y;
   wherein a side chain of an amino acid of the peptide is optionally conjugated to a lipophilic substituent or polymeric moiety;
   wherein the peptide of Formula I optionally has a disulfide bond formed between the thiol groups of two cysteine residues; and
   wherein Ida is iminodiacetic acid, pGlu is pyroglutamic acid, bhAsp is β-homoaspartic acid, and bhPro is β-homoproline.
10. The method of embodiment 9, wherein R1 is hydrogen, isovaleric acid, isobutyric acid or acetyl.
11. The method of embodiment 9 or embodiment 10, wherein X is an amino acid sequence of Formula IV:

   X1-Thr-His-X4-X5-X6-X7-X8-Phe-X10 (IV)

   wherein
   X1 is Asp, Ida, pGlu, bhAsp or absent;
   X4 is Phe or Dpa;
   X5 is Pro or bhPro;
   X6 is Ile, Cys, or Arg;
   X7 is Cys, Ile, Leu, or Val;
   X8 is Ile, Lys, Glu, Phe, Gln, or Arg; and
   X10 is Lys or absent.
12. The method of embodiment 10 or embodiment 11, wherein X is an amino acid sequence of Formula V:

   X1-Thr-His-X4-X5-Cys-Ile-X8-Phe-X10 (V)

   wherein
   X1 is Asp, Ida, pGlu, bhAsp, or absent;
   X4 is Phe or Dpa;
   X5 is Pro or bhPro;
   X8 is Ile, Lys, Glu, Phe, Gln or Arg; and
   X10 is Lys or absent.
13. The method of embodiment 9, wherein the peptide has Formula VI:

   R¹-X-Y-R² (VI)

   or a pharmaceutically acceptable salt thereof, wherein:
   R¹ is hydrogen, isovaleric acid, isobutyric acid, or acetyl;
   R² is NH₂ or OH;
   X is an amino acid sequence of Formula VII:

      X1-Thr-His-X4-X5-Cys-Ile-X8-Phe-X10 (VII)
   wherein
   X1 is Asp, Ida, pGlu, bhAsp, or absent;
   X4 is Phe or Dpa;
   X5 is Pro or bhPro;
   X8 is Ile, Lys, Glu, Phe, Gln, or Arg; and
   X10 is Lys or absent;
   wherein Y is an amino acid sequence of Formula VIII:

      Y1-Pro-Y3-Ser-Y5-Y6-Y7-Y8-Cys-Y10 (VIII)
   wherein
   Y1 is Gly, Glu, Val, or Lys;
   Y3 is Arg or Lys;
   Y5 is Arg or Lys;
   Y6 is Gly, Ser, Lys, Ile, or Arg;
   Y7 is Trp or absent;
   Y8 is Val, Thr, Asp, Glu, or absent; and
   Y10 is Lys or absent;
   wherein the peptide has a disulfide bond formed between the thiol groups of two cysteine residues;
   wherein the peptide is optionally PEGylated on R¹, X, or Y;
   wherein a side chain of an amino acid of the peptide is optionally conjugated to a lipophilic substituent or polymeric moiety; and
   wherein Ida is iminodiacetic acid; pGlu is pyroglutamic acid; bhAsp is β-homoaspartic acid; and bhPro is β-homoproline.
14. The method of any one of embodiments 9-13, wherein the peptide has one of the following sequences:
   DTHFPICIFGPRSKGWVC (SEQ ID NO:46);
   DTHFPCIIFGPRSKGWVCK (SEQ ID NO:47);
   DTHFPCIIFEPRSKGWVCK (SEQ ID NO:48);
   DTHFPCIIFGPRSKGWACK (SEQ ID NO:49);
   DTHFPCIIFGPRSKGWVCKK (SEQ ID NO:50);
   DTHFPCIIFVCHRPKGCYRRVCR (SEQ ID NO:51);
   DTHFPCIKFGPRSKGWVCK (SEQ ID NO:52);
   DTHFPCIKFKPRSKGWVCK (SEQ ID NO:53);
   DTHFPCIIFGPRSRGWVCK (SEQ ID NO:54);
   DTHFPCIKFGPKSKGWVCK (SEQ ID NO:55);
   DTHFPCIKFEPRSKGCK (SEQ ID NO:56);
   DTHFPCIKFEPKSKGWECK (SEQ ID NO:57);
   DTHFPCIKFEPRSKKCK (SEQ ID NO:58);
   DTHFPCIKFEPRSKGCKK (SEQ ID NO:59);
   DTHFPCIKFKPRSKGCK (SEQ ID NO:60);
   DTHFPCIKFEPKSKGCK (SEQ ID NO:61);
   DTHFPCIKF (SEQ ID NO:62);
   DTHFPCIIF (SEQ ID NO:63); or
   DTKFPCIIF (SEQ ID NO:64),

   wherein said peptide is optionally PEGylated on R1, X, or Y; and
   wherein a side chain of an amino acid of the peptide is optionally conjugated to a lipophilic substituent or polymeric moiety.
15. The method of any one of embodiments 9-13, wherein the peptide has one of the following sequences or structures:
   Isovaleric acid-DTHFPICIFGPRSKGWVC-NH₂ (Compound 1; SEQ ID NO:1);
   Isovaleric acid-DTHFPCIIFGPRSKGWVCK-NH₂ (Compound 2; SEQ ID NO:2);
   Isovaleric acid-DTHFPCIIFEPRSKGWVCK-NH₂ (Compound 3; SEQ ID NO:3);
   Isovaleric acid-DTHFPCIIFGPRSKGWACK-NH₂ (Compound 4; SEQ ID NO:4);
   Isovaleric acid-DTHFPCIIFGPRSKGWVCKK-NH₂ (Compound 5; SEQ ID NO:5);
   Isovaleric acid-DTHFPCIIFVCHRPKGCYRRVCR-NH₂ (Compound 6; SEQ ID NO:6);
   Isovaleric acid-DTHFPCI(K(PEG8))FGPRSKGWVCK-NH₂ (Compound 7; SEQ ID NO:7);
   Isovaleric acid-DTHFPCIKF(K(PEG8))PRSKGWVCK-NH₂ (Compound 8; SEQ ID NO:8);
   Isovaleric acid-DTHFPICIFGPRS(K(PEG8))GWVC-NH₂ (Compound 9; SEQ ID NO:9);
   Isovaleric acid-DTHFPICIFGPRS(K(PEG4))GWVC-NH₂ (Compound 10; SEQ ID NO:10)_{;}
   Isovaleric acid-DTHFPCIIFGPRSRGWVC(K(PEG8))-NH₂ (Compound 11; SEQ ID NO: 11);
   Isovaleric acid-DTHFPCIIFGPRSRGWVC(K(PEG4))-NH₂(Compound 12; SEQ ID NO:12) _{;}
   Isovaleric acid-DTHFPCIIFGPRSRGWVC(K(PEG2))-NH₂ (Compound 13: SEQ ID NO:13) ;
   Isovaleric acid-DTHFPCI(K(Palm))FGPRSKGWVCK-NH₂ (Compound 14; SEQ ID NO:14)
   Isovaleric acid-DTHFPCIKF)K(Palm))PRSKGWVCK-NH₂(Compound 15; SEQ ID NO:15) ;
   Isovaleric acid-DTHFPCIKFGP(K(Palm))SKGWVCK-NH₂(Compound 16; SEQ ID NO:16) ;
   Isovaleric acid-DTHFPCIKFGPRS(K(Palm))GWVCK-NH₂ (Compound 17; SEQ ID NO:17)
   Isovaleric acid-DTHFPCIKFGPRSKGWVC(K(Palm))NH₂ (Compound 18; SEQ ID NO:18) ;
   Isovaleric acid-DTHFPCI(K(PEG3-Palm))FGPRSKGWVCK-NH₂ (Compound 19; SEQ ID NO:19);
   Isovaleric acid-DTHFPCIKF(K(PEG3-Palm))PRSKGWVCK-NH₂ (Compound 20; SEQ ID NO:20) ;
   Isovaleric acid-DTHFPCIKFGP(K(PEG3-Palm))SKGWVCK-NH₂ (Compound 21; SEQ ID NO:21) ;
   Isovaleric acid-DTHFPCIKFGPRS(K(PEG3-Palm))GWVCK-NH₂ (Compound 22; SEQ ID NO:22);
   Isovaleric acid-DTHFPCIKFGPRSKGWVC(K(PEG3-Palm))-NH₂ (Compound 23; SEQ ID NO:23);
   Isovaleric acid-DTHFPCIKFGPRSKGWVC(K(PEG8))-NH₂ (Compound 24; SEQ ID NO:24);
   Isovaleric acid-DTHFPCI(K(isoGlu-Palm))FEPRSKGCK-NH₂ (Compound 25; SEQ ID NO:25);
   Isovaleric acid-DTHFPCIKF-K(isoGlu-Palm)-PRSKGCK-NH₂ (Compound 26; SEQ ID NO:26);
   Isovaleric acid-DTHFPCIKFEP(K(isoGlu-Palm))SKGCK-NH₂ (Compound 27; SEQ ID NO:27);
   Isovaleric acid-DTHFPCIKFEP(K(isoGlu-Palm))SKGWECK-NH₂ (Compound 28; SEQ ID NO:28);
   Isovaleric acid-DTHFPCIKFEPRS(K(isoGlu-Palm))GCK-NH₂ (Compound 29; SEQ ID NO:29);
   Isovaleric acid-DTHFPCIKFEPRSK(K(isoGlu-Palm))CK-NH₂ (Compound 30; SEQ ID NO:30);
   Isovaleric acid-DTHFPCIKFEPRSKGCK(K(isoGlu-Palm))-NH₂ (Compound 31; SEQ ID NO:31)_{;}
   Isovaleric acid-DTHFPCI-K(Dapa-Palm)-FEPRSKGCK-NH₂ (Compound 32; SEQ ID NO:32);
   Isovaleric acid-DTHFPCIK(F(Dapa-Palm))PRSKGCK-NH₂ (Compound 33; SEQ ID NO:33);
   Isovaleric acid-DTHFPCIKFEP(K(Dapa-Palm))SKGCK-NH₂ (Compound 34; SEQ ID NO:34);
   Isovaleric acid-DTHFPCIKFEPRS(K(Dapa-Palm))GCK-NH₂ (Compound 35; SEQ ID NO:35);
   Isovaleric acid-DTHFPCIKFEPRSK(K(Dapa-Palm))CK-NH₂ (Compound 36; SEQ ID NO:36);
   Isovaleric acid-DTHFPCIKFEPRSKGC(K(Dapa-Palm))K-NH₂ (Compound 37; SEQ ID NO:37);
   Isovaleric acid-DTHFPCIKFEPRSKGC(K(Dapa-Palm))-NH₂ (Compound 38; SEQ ID NO:38);
   Isovaleric acid-DTHFPCIKF(K(PEG11-Palm))PRSK[Sar]CK-NH₂ (Compound 39; SEQ ID NO:39);
   Isolvaleric acid-DTHFPCIKF-NH₂ (Compound 40; SEQ ID NO:40);
   Hy-DTHFPCIKF-NH₂ (Compound 41; SEQ ID NO:41);
   Isolvaleric acid-DTHFPCIIF-NH₂ (Compound 42; SEQ ID NO:42);
   Hy-DTHFPCIIKF-NH₂ (Compound 43; SEQ ID NO:43);
   Isovaleric acid-DTKFPCIIF-NH₂ (Compound 44; SEQ ID NO:44); or
   Hy-DTKFPCIIF-NH₂ (Compound 45; SEQ ID NO:45),
   optionally, wherein the peptide has a disulfide bond formed between the thiol groups of two cysteine residues.
16. The method of embodiment 9 or embodiment 10, wherein the peptide is: Isovaleric acid-DTHFPCIIFGPRSKGWVCK-NH₂ (Compound 2; SEQ ID NO:2),
   optionally wherein the peptide has a disulfide bond formed between the thiol groups of two cysteine residues.
17. The method of embodiment 9 or embodiment 10, wherein the peptide is: Isovaleric acid-DTHFPCIIFEPRSKGWVCK-NH₂ (Compound 3; SEQ ID NO:3),
   optionally wherein the peptide has a disulfide bond formed between the thiol groups of two cysteine residues.
18. The method of embodiment 9 or embodiment 10, wherein the peptide is: Isovaleric acid-DTHFPCI(K(PEG8))FGPRSKGWVCK-NH₂ (Compound 7; SEQ ID NO:7),
   optionally wherein the peptide has a disulfide bond formed between the thiol groups of two cysteine residues.
19. The method of embodiment 9 or embodiment 10, wherein the peptide is: Isovaleric acid-DTHFPCIKF(K(PEG8))PRSKGWVCK-NH₂ (Compound 8; SEQ ID NO:8),
   optionally wherein the peptide has a disulfide bond formed between the thiol groups of two cysteine residues.
20. The method of embodiment 9 or embodiment 10, wherein the peptide is: Isovaleric acid-DTHFPCIIFGPRSRGWVC(K(PEG8))-NH₂ (Compound 11; SEQ ID NO:11),
   optionally wherein the peptide has a disulfide bond formed between the thiol groups of two cysteine residues.
21. The method of embodiment 9 or embodiment 10, wherein the peptide is: Isovaleric acid-DTHFPCI(K(Palm))FGPRSKGWVCK-NH₂ (Compound 14; SEQ ID NO:14),
   optionally wherein the peptide has a disulfide bond formed between the thiol groups of two cysteine residues.
22. The method of embodiment 9 or embodiment 10, wherein the peptide is: Isovaleric acid-DTHFPCIKF(K(Palm))PRSKGWVCK-NH₂ (Compound 15; SEQ ID NO:15),
   optionally wherein the peptide has a disulfide bond formed between the thiol groups of two cysteine residues.
23. The method of embodiment 9 or embodiment 10, wherein the peptide is: Isovaleric acid-DTHFPCIKFGP(K(Palm))SKGWVCK-NH₂ (Compound 16; SEQ ID NO:16),
   optionally wherein the peptide has a disulfide bond formed between the thiol groups of two cysteine residues.
24. The method of embodiment 9 or embodiment 10, wherein the peptide is: Isovaleric acid-DTHFPCIKFGPRSKGWVC(K(Palm))-NH₂ (Compound 18; SEQ ID NO:18),
   optionally wherein the peptide has a disulfide bond formed between the thiol groups of two cysteine residues.
25. The method of embodiment 9 or embodiment 10, wherein the peptide is: Isovaleric acid-DTHFPCI(K(PEG3-Palm))FGPRSKGWVCK-NH₂ (Compound 19; SEQ ID NO:19), optionally wherein the peptide has a disulfide bond formed between the thiol groups of two cysteine residues.
26. The method of embodiment 9 or embodiment 10, wherein the peptide is: Isovaleric acid-DTHFPCIKF(K(PEG3-Palm))PRSKGWVCK-NH₂ (Compound 20; SEQ ID NO:20), optionally wherein the peptide has a disulfide bond formed between the thiol groups of two cysteine residues.
27. The method of embodiment 9 or embodiment 10, wherein the peptide is: Isovaleric acid-DTHFPCIKFGP(K(PEG3-Palm))SKGWVCK-NH₂ (Compound 21; SEQ ID NO:21), optionally wherein the peptide has a disulfide bond formed between the thiol groups of two cysteine residues.
28. The method of embodiment 9 or embodiment 10, wherein the peptide is: Isovaleric acid-DTHFPCIKFGPRS(K(PEG3-Palm))GWVCK-NH₂ (Compound 22; SEQ ID NO:22), optionally wherein the peptide has a disulfide bond formed between the thiol groups of two cysteine residues.
29. The method of embodiment 9 or embodiment 10, wherein the peptide is: Isovaleric acid-DTHFPCIKFGPRSKGWVC(K(PEG3-Palm))-NH₂ (Compound 23; SEQ ID NO:23), optionally wherein the peptide has a disulfide bond formed between the thiol groups of two cysteine residues.
30. The method of embodiment 9 or embodiment 10, wherein the peptide is: Isovaleric acid-DTHFPCIKFGPRSKGWVC(K(PEG8))-NH₂ (Compound 24; SEQ ID NO:24),
   optionally wherein the peptide has a disulfide bond formed between the thiol groups of two cysteine residues.
31. The method of embodiment 9 or embodiment 10, wherein the peptide is: Isovaleric acid-DTHFPCI(K(isoGlu-Palm))FEPRSKGCK-NH₂ (Compound 25; SEQ ID NO:25), optionally wherein the peptide has a disulfide bond formed between the thiol groups of two cysteine residues.
32. The method of embodiment 9 or embodiment 10, wherein the peptide is: Isovaleric acid-DTHFPCIKF(K(isoGlu-Palm))PRSKGCK-NH₂ (Compound 26; SEQ ID NO:26), optionally wherein the peptide has a disulfide bond formed between the thiol groups of two cysteine residues.
33. The method of embodiment 9 or embodiment 10, wherein the peptide is: Isovaleric acid-DTHFPCIKFEP(K(isoGlu-Palm))SKGCK-NH₂ (Compound 27; SEQ ID NO:27), optionally wherein the peptide has a disulfide bond formed between the thiol groups of two cysteine residues.
34. The method of embodiment 9 or embodiment 10, wherein the peptide is: Isovaleric acid-DTHFPCIKFEPRS(K(isoGlu-Palm))GCK-NH₂ (Compound 28; SEQ ID NO:28), optionally wherein the peptide has a disulfide bond formed between the thiol groups of two cysteine residues.
35. The method of embodiment 9 or embodiment 10, wherein the peptide is: Isovaleric acid-DTHFPCI(K(Dapa-Palm))FEPRSKGCK-NH₂ (Compound 32; SEQ ID NO:32),
   optionally wherein the peptide has a disulfide bond formed between the thiol groups of two cysteine residues.
36. The method of embodiment 9 or embodiment 10, wherein the peptide is: Isovaleric acid-DTHFPCIKFEP(K(Dapa-Palm))SKGCK-NH₂ (Compound 34; SEQ ID NO:34),
   optionally wherein the peptide has a disulfide bond formed between the thiol groups of two cysteine residues.
37. The method of embodiment 9 or embodiment 10, wherein the peptide is selected from the group consisting of:
   (a) 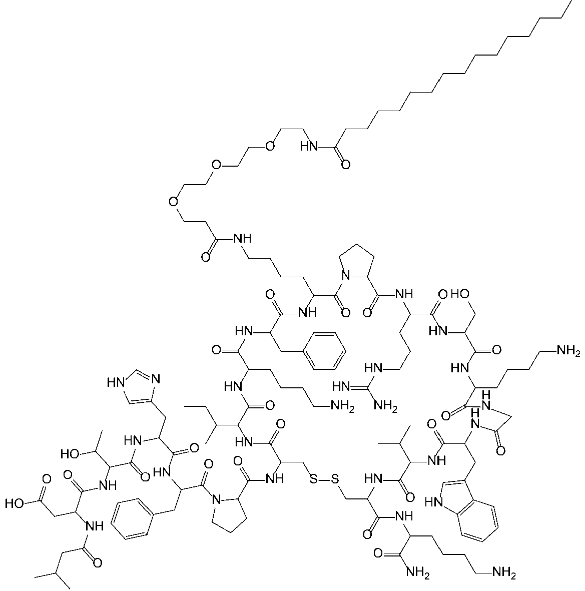 Isovaleric acid-DTHFPCIKF(K(PEG3-Palm))PRSKGWVCK-NH₂ (Compound 20; SEQ ID NO:20);
   (b) 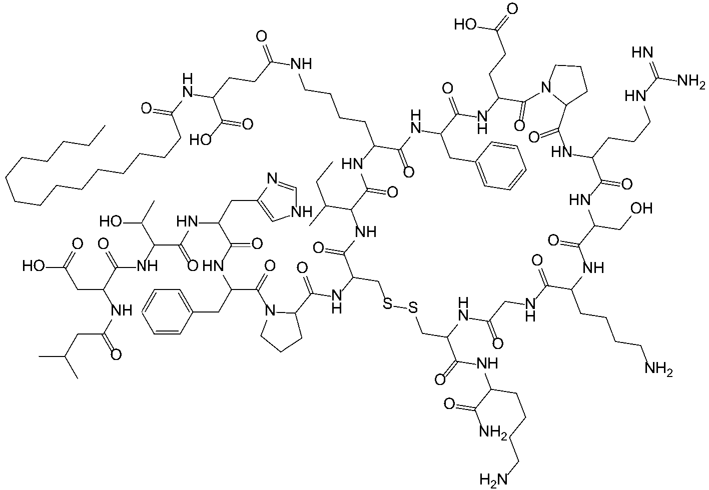 Isovaleric acid-DTHFPCI(K(isoGlu-Palm))FEPRSKGCK-NH₂ (Compound 25; SEQ ID NO:25);
   (c) 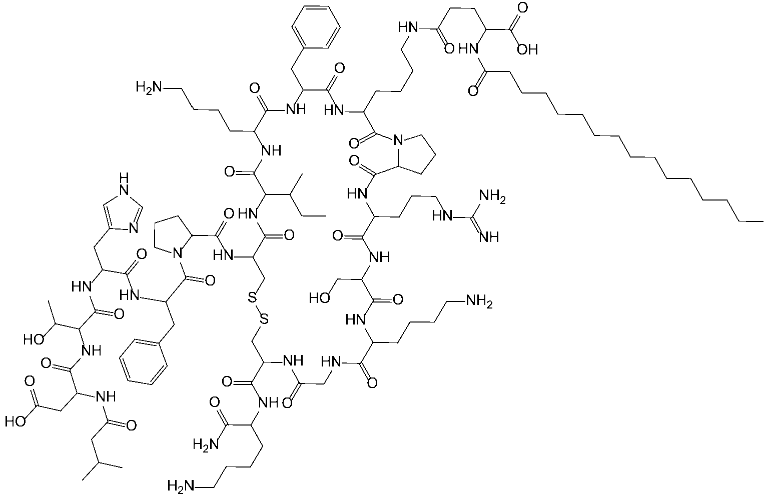 Isovaleric acid-DTHFPCIKF(K(isoGlu-Palm))PRSKGCK-NH₂ (Compound 26; SEQ ID NO:26);
   (d) 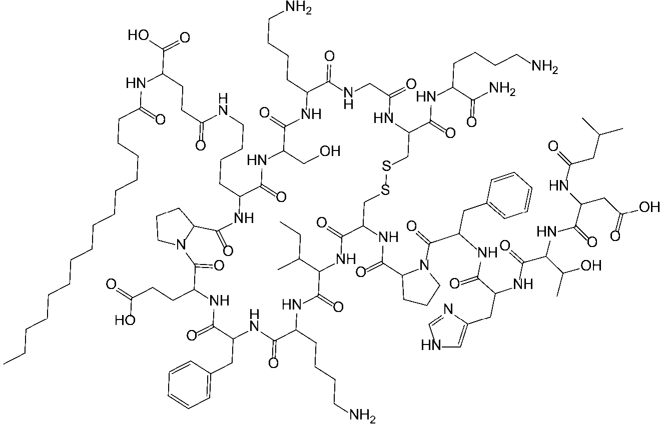 Isovaleric acid-DTHFPCIKFEP(K(isoGlu-Palm))SKGCK-NH₂ (Compound 27; SEQ ID NO:27); and
   (e) 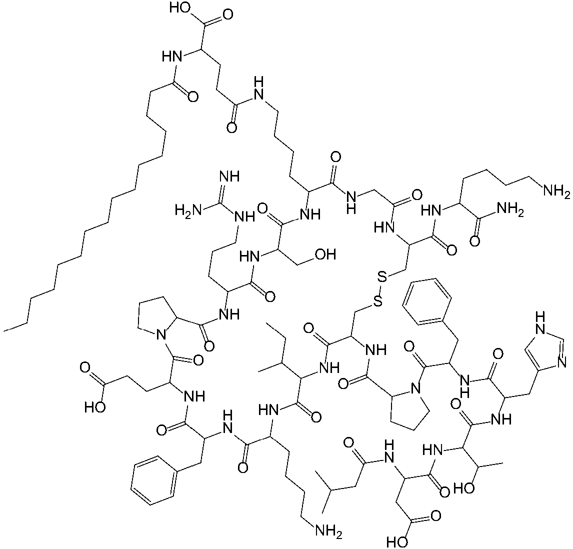 Isovaleric acid-DTHFPCIKFEPRS(K(isoGlu-Palm))GCK-NH₂ (Compound 28; SEQ ID NO:28),
   wherein the amino acids are L-amino acids.
38. The method of any one of embodiments 1-37, wherein the method comprises measuring TSAT and/or serum iron level in the subject before and after the hepcidin mimetic is administered to the subject, optionally wherein the TSAT level is measured at trough level of the hepcidin mimetic following administration to the subject.
39. The method of any one of embodiments 1-38, wherein the hepcidin mimetic is administered to the subject subcutaneously.
40. The method of any one of embodiments 1-39, wherein the subject received phlebotomies for at least six months prior to treatment, optionally with a phlebotomy frequency of 0.25 - 1 phlebotomy per month.
41. The method of embodiment 40, wherein during the treatment, the subject required substantially fewer or no phlebotomies, optionally with a phlebotomy frequency of less than 0.1, less than 0.05, or no phlebotomies per month.
42. A method for treating hereditary hemochromatosis in a human subject, comprising administering to the subject an effective amount of Compound 25 having the formula: 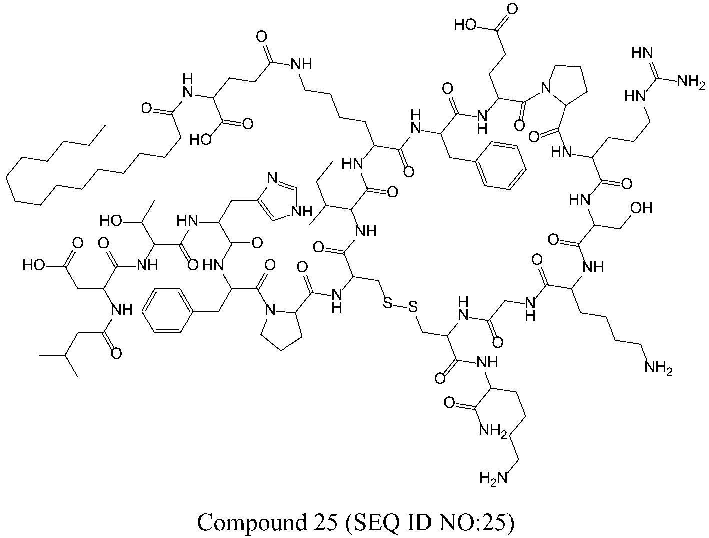 or a pharmaceutically acceptable salt thereof, wherein the effective amount comprises a dose in the range of about 5 mg to about 40 mg, and optionally wherein the subject is administered different doses during different time periods over a course of treatment.
43. A method for treating hereditary hemochromatosis in a human subject, comprising administering to the subject an effective amount of a peptide having the sequence: Isovaleric acid-DTHFPCI(K(isoGlu-Palm))FEPRSKGCK-NH₂ (SEQ ID NO:25), or a pharmaceutically acceptable salt thereof, wherein the thiol groups of two cysteine residues in the peptide optionally form a disulfide bond, wherein the effective amount comprises a dose in the range of about 5 mg to about 40 mg, and optionally wherein the subject is administered different doses during different time periods over a course of treatment.
44. A method for treating hereditary hemochromatosis arthropathy or joint pain associated with hereditary hemochromatosis arthropathy in a human subject, comprising administering to the subject an effective amount of a hepcidin mimetic.
45. The method of embodiment 44, wherein the effective amount comprises a dose in the range of about 5 mg to about 40 mg, and optionally wherein the subject is administered different doses during different time periods over a course of treatment.
46. The method of embodiment 44 or 45, wherein the hepcidin mimetic is compound 25.

## Claims

1. A hepcidin mimetic for use in a method for treating hereditary hemochromatosis, hereditary hemochromatosis arthropathy, or joint pain associated with hereditary hemochromatosis arthropathy in a human subject, the method comprising administering to the subject an effective amount of the hepcidin mimetic, wherein the effective amount comprises a dose in the range of 5 mg to 40 mg.

2. The hepcidin mimetic for use of claim 1, wherein the subject is administered different doses during different time periods over a course of treatment.

3. The hepcidin mimetic for use of claim 1 or claim 2, wherein the subject is administered the effective amount of the hepcidin mimetic about once a week or about twice a week for at least some time period during the course of treatment;
optionally wherein the subject is administered 5 mg to 20 mg of the hepcidin mimetic about twice a week for at least some time period during the course of treatment; or wherein the subject is administered 10 mg to 40 mg of the hepcidin mimetic about once a week for at least some time period during the course of treatment.

4. The hepcidin mimetic for use of any one of claims 1-3, wherein hepcidin mimetic is a peptide having Formula I:
R¹-X-Y-R² (I)
or a pharmaceutically acceptable salt or solvate thereof,
wherein
R1 is C1-C20 alkanoyl, a hydrogen, a C1-C6 alkyl, a C6-C12 aryl, or pGlu;
R2 is NH₂ or OH;
X is an amino acid sequence of Formula II:
X1-X2-X3-X4-X5-X6-X7-X8-X9-X10 (II)
wherein
X1 is Asp, Ala, Ida, pGlu, bhAsp, Leu, D-Asp, or absent;
X2 is Thr, Ala, or D-Thr;
X3 is His, Lys, D-His, or Lys;
X4 is Phe, Ala, Dpa, or D-Phe;
X5 is Pro, Gly, Arg, Lys, Ala, D-Pro, or bhPro;
X6 is Cys, Ile, Arg, Lys, D-Ile, or D-Cys;
X7 is Ile, Cys Leu, Val, Phe, D-Ile, or D-Cys;
X8 is Lys, Ile, Arg, Phe, Gln, Glu, Val, Leu, or D-Ile;
X9 is Phe or bhPhe; and
X10 is absent, Lys, or Phe;
wherein if Y is absent, X7 is Ile; and
Y is an amino acid sequence of Formula III:
Y1-Y2-Y3-Y4-Y5-Y6-Y7-Y8-Y9-Y10-Y11-Y12-Y13-Y14-Y15 (III)
wherein
Y1 is Glu, Gly, Cys, Ala, Phe, Pro, Lys, D-Pro, Val, Ser, or absent;
Y2 is Pro, Ala, Cys, Gly, or absent;
Y3 is Arg, Lys, Pro, Gly, His, Ala, Trp, or absent;
Y4 is Ser, Arg, Gly, Trp, Ala, His, Tyr, or absent;
Y5 is Lys, Met, Arg, Ala, or absent;
Y6 is Gly, Ser, Lys, Ile, Ala, Pro, Val, or absent;
Y7 is absent, Trp, Lys, Gly, Ala, Ile, or Val;
Y8 is absent, Val, Thr, Gly, Cys, Met, Tyr, Ala, Glu, Lys, Asp, or Arg;
Y9 is Cys, Tyr, or absent;
Y10 is Lys, Met, Arg, Tyr, or absent;
Y11 is absent, Arg, Met, Cys, or Lys;
Y12 is absent, Arg, Lys, or Ala;
Y13 is absent, Arg, Cys, Lys, or Val;
Y14 is absent, Arg, Lys, Pro, Cys, or Thr; and
Y15 is absent, Thr, or Arg;
wherein the peptide of Formula I is optionally PEGylated on X, R1 or Y;
wherein a side chain of an amino acid of the peptide is optionally conjugated to a lipophilic substituent or polymeric moiety;
wherein the peptide of Formula I optionally has a disulfide bond formed between the thiol groups of two cysteine residues; and
wherein Ida is iminodiacetic acid, pGlu is pyroglutamic acid, bhAsp is β-homoaspartic acid, and bhPro is β-homoproline;
optionally wherein R1 is isovaleric acid, hydrogen, isobutyric acid or acetyl.

5. The hepcidin mimetic for use of claim 4, wherein:
(a) X is an amino acid sequence of Formula IV:
X1-Thr-His-X4-X5-X6-X7-X8-Phe-X10 (IV)
wherein
X1 is Asp, Ida, pGlu, bhAsp or absent;
X4 is Phe or Dpa;
X5 is Pro or bhPro;
X6 is Cys, Ile, or Arg;
X7 is Ile, Cys, Leu, or Val;
X8 is Lys, Ile, Glu, Phe, Gln, or Arg; and
X10 is absent or Lys;
or
(b) X is an amino acid sequence of Formula V:
X1-Thr-His-X4-X5-Cys-Ile-X8-Phe-X10 (V)
wherein
X1 is Asp, Ida, pGlu, bhAsp, or absent;
X4 is Phe or Dpa;
X5 is Pro or bhPro;
X8 is Lys, Ile, Glu, Phe, Gln or Arg; and
X10 is absent or Lys.

6. The hepcidin mimetic for use of claim 4, wherein the peptide has Formula VI:
R¹-X-Y-R² (VI)
or a pharmaceutically acceptable salt thereof, wherein:
R¹ is isovaleric acid, hydrogen, isobutyric acid, or acetyl;
R² is NH₂ or OH;
X is an amino acid sequence of Formula VII:
X1-Thr-His-X4-X5-Cys-Ile-X8-Phe-X10 (VII)
wherein
X1 is Asp, Ida, pGlu, bhAsp, or absent;
X4 is Phe or Dpa;
X5 is Pro or bhPro;
X8 is Lys, Ile, Glu, Phe, Gln, or Arg; and
X10 is absent or Lys;
wherein Y is an amino acid sequence of Formula VIII:
Y1-Pro-Y3-Ser-Y5-Y6-Y7-Y8-Cys-Y 10 (VIII)
wherein
Y1 is Glu, Gly, Val, or Lys;
Y3 is Arg or Lys;
Y5 is Lys or Arg;
Y6 is Gly, Ser, Lys, Ile, or Arg;
Y7 is absent or Trp;
Y8 is absent, Val, Thr, Asp, or Glu; and
Y10 is Lys or absent;
wherein the peptide has a disulfide bond formed between the thiol groups of two cysteine residues;
wherein the peptide is optionally PEGylated on R¹, X, or Y; and
wherein a side chain of an amino acid of the peptide is optionally conjugated to a lipophilic substituent or polymeric moiety.

7. The hepcidin mimetic for use of any one of claims 4-6, wherein:
(a) the peptide has one of the following sequences or structures:
Isovaleric acid-DTHFPCI(K(isoGlu-Palm))FEPRSKGCK-NH₂ (Compound 25; SEQ ID NO:25);
Isovaleric acid-DTHFPICIFGPRSKGWVC-NH₂ (Compound 1; SEQ ID NO:1);
Isovaleric acid-DTHFPCIIFGPRSKGWVCK-NH₂ (Compound 2; SEQ ID NO:2);
Isovaleric acid-DTHFPCIIFEPRSKGWVCK-NH₂ (Compound 3; SEQ ID NO:3);
Isovaleric acid-DTHFPCIIFGPRSKGWACK-NH₂ (Compound 4; SEQ ID NO:4);
Isovaleric acid-DTHFPCIIFGPRSKGWVCKK-NH₂ (Compound 5; SEQ ID NO:5);
Isovaleric acid-DTHFPCIIFVCHRPKGCYRRVCR-NH₂ (Compound 6; SEQ ID NO:6);
Isovaleric acid-DTHFPCI(K(PEG8))FGPRSKGWVCK-NH₂ (Compound 7; SEQ ID NO:7);
Isovaleric acid-DTHFPCIKF(K(PEG8))PRSKGWVCK-NH₂ (Compound 8; SEQ ID NO:8);
Isovaleric acid-DTHFPICIFGPRS(K(PEG8))GWVC-NH₂ (Compound 9; SEQ ID NO:9);
Isovaleric acid-DTHFPICIFGPRS(K(PEG4))GWVC-NH₂ (Compound 10; SEQ ID NO:10);
Isovaleric acid-DTHFPCIIFGPRSRGWVC(K(PEG8))-NH₂ (Compound 11; SEQ ID NO:11);
Isovaleric acid-DTHFPCIIFGPRSRGWVC(K(PEG4))-NH₂(Compound 12; SEQ ID NO:12);
Isovaleric acid-DTHFPCIIFGPRSRGWVC(K(PEG2))-NH₂ (Compound 13: SEQ ID NO:13);
Isovaleric acid-DTHFPCI(K(Palm))FGPRSKGWVCK-NH₂ (Compound 14; SEQ ID NO:14);
Isovaleric acid-DTHFPCIKF)K(Palm))PRSKGWVCK-NH₂(Compound 15; SEQ ID NO:15);
Isovaleric acid-DTHFPCIKFGP(K(Palm))SKGWVCK-NH₂(Compound 16; SEQ ID NO:16);
Isovaleric acid-DTHFPCIKFGPRS(K(Palm))GWVCK-NH₂ (Compound 17; SEQ ID NO: 17);
Isovaleric acid-DTHFPCIKFGPRSKGWVC(K(Palm))NH₂ (Compound 18; SEQ ID NO:18);
Isovaleric acid-DTHFPCI(K(PEG3-Palm))FGPRSKGWVCK-NH₂ (Compound 19; SEQ ID NO: 19);
Isovaleric acid-DTHFPCIKF(K(PEG3-Palm))PRSKGWVCK-NH₂ (Compound 20; SEQ ID NO:20);
Isovaleric acid-DTHFPCIKFGP(K(PEG3-Palm))SKGWVCK-NH₂ (Compound 21; SEQ ID NO:21);
Isovaleric acid-DTHFPCIKFGPRS(K(PEG3-Palm))GWVCK-NH₂ (Compound 22; SEQ ID NO:22);
Isovaleric acid-DTHFPCIKFGPRSKGWVC(K(PEG3-Palm))-NH₂ (Compound 23; SEQ ID NO:23);
Isovaleric acid-DTHFPCIKFGPRSKGWVC(K(PEG8))-NH₂ (Compound 24; SEQ ID NO:24);
Isovaleric acid-DTHFPCIKF-K(isoGlu-Palm)-PRSKGCK-NH₂ (Compound 26; SEQ ID NO:26);
Isovaleric acid-DTHFPCIKFEP(K(isoGlu-Palm))SKGCK-NH₂ (Compound 27; SEQ ID NO:27);
Isovaleric acid-DTHFPCIKFEP(K(isoGlu-Palm))SKGWECK-NH₂ (Compound 28; SEQ ID NO:28);
Isovaleric acid-DTHFPCIKFEPRS(K(isoGlu-Palm))GCK-NH₂ (Compound 29; SEQ ID NO:29);
Isovaleric acid-DTHFPCIKFEPRSK(K(isoGlu-Palm))CK-NH₂ (Compound 30; SEQ ID NO:30);
Isovaleric acid-DTHFPCIKFEPRSKGCK(K(isoGlu-Palm))-NH₂ (Compound 31; SEQ ID NO:31);
Isovaleric acid-DTHFPCI-K(Dapa-Palm)-FEPRSKGCK-NH₂ (Compound 32; SEQ ID NO:32);
Isovaleric acid-DTHFPCIK(F(Dapa-Palm))PRSKGCK-NH₂ (Compound 33; SEQ ID NO:33);
Isovaleric acid-DTHFPCIKFEP(K(Dapa-Palm))SKGCK-NH₂ (Compound 34; SEQ ID NO:34);
Isovaleric acid-DTHFPCIKFEPRS(K(Dapa-Palm))GCK-NH₂ (Compound 35; SEQ ID NO:35);
Isovaleric acid-DTHFPCIKFEPRSK(K(Dapa-Palm))CK-NH₂ (Compound 36; SEQ ID NO:36);
Isovaleric acid-DTHFPCIKFEPRSKGC(K(Dapa-Palm))K-NH₂ (Compound 37; SEQ ID NO:37);
Isovaleric acid-DTHFPCIKFEPRSKGC(K(Dapa-Palm))-NH₂ (Compound 38; SEQ ID NO:38);
Isovaleric acid-DTHFPCIKF(K(PEG11-Palm))PRSK[Sar]CK-NH₂ (Compound 39; SEQ ID NO:39);
Isolvaleric acid-DTHFPCIKF-NH₂ (Compound 40; SEQ ID NO:40); Hy-DTHFPCIKF-NH₂ (Compound 41; SEQ ID NO:41);
Isolvaleric acid-DTHFPCIIF-NH₂ (Compound 42; SEQ ID NO:42); Hy-DTHFPCIIKF-NH₂ (Compound 43; SEQ ID NO:43);
Isovaleric acid-DTKFPCIIF-NH₂ (Compound 44; SEQ ID NO:44); or Hy-DTKFPCIIF-NH₂ (Compound 45; SEQ ID NO:45);
or
(b) the peptide has one of the following sequences:
DTHFPCIKFEPRSKGCK (SEQ ID NO:56);
DTHFPICIFGPRSKGWVC (SEQ ID NO:46);
DTHFPCIIFGPRSKGWVCK (SEQ ID NO:47);
DTHFPCIIFEPRSKGWVCK (SEQ ID NO:48);
DTHFPCIIFGPRSKGWACK (SEQ ID NO:49);
DTHFPCIIFGPRSKGWVCKK (SEQ ID NO:50);
DTHFPCIIFVCHRPKGCYRRVCR (SEQ ID NO:51);
DTHFPCIKFGPRSKGWVCK (SEQ ID NO:52);
DTHFPCIKFKPRSKGWVCK (SEQ ID NO:53);
DTHFPCIIFGPRSRGWVCK (SEQ ID NO:54);
DTHFPCIKFGPKSKGWVCK (SEQ ID NO:55);
DTHFPCIKFEPKSKGWECK (SEQ ID NO:57);
DTHFPCIKFEPRSKKCK (SEQ ID NO:58);
DTHFPCIKFEPRSKGCKK (SEQ ID NO:59);
DTHFPCIKFKPRSKGCK (SEQ ID NO:60);
DTHFPCIKFEPKSKGCK (SEQ ID NO:61);
DTHFPCIKF (SEQ ID NO:62);
DTHFPCIIF (SEQ ID NO:63); or
DTKFPCIIF (SEQ ID NO:64),
wherein said peptide is optionally PEGylated on R1, X, or Y; and
wherein a side chain of an amino acid of the peptide is optionally conjugated to a lipophilic substituent or polymeric moiety.

8. The hepcidin mimetic for use of claim 4, wherein the peptide is:
(a) Isovaleric acid-DTHFPCI(K(isoGlu-Palm))FEPRSKGCK-NH₂ (Compound 25; SEQ ID NO:25);
(b) Isovaleric acid-DTHFPCIIFGPRSKGWVCK-NH₂ (Compound 2; SEQ ID NO:2), optionally wherein the peptide has a disulfide bond formed between the thiol groups of two cysteine residues;
(c) Isovaleric acid-DTHFPCIIFEPRSKGWVCK-NH₂ (Compound 3; SEQ ID NO:3), optionally wherein the peptide has a disulfide bond formed between the thiol groups of two cysteine residues;
(d) Isovaleric acid-DTHFPCI(K(PEG8))FGPRSKGWVCK-NH₂ (Compound 7; SEQ ID NO:7), optionally wherein the peptide has a disulfide bond formed between the thiol groups of two cysteine residues;
(e) Isovaleric acid-DTHFPCIKF(K(PEG8))PRSKGWVCK-NH₂ (Compound 8; SEQ ID NO:8), optionally wherein the peptide has a disulfide bond formed between the thiol groups of two cysteine residues;
(f) Isovaleric acid-DTHFPCIIFGPRSRGWVC(K(PEG8))-NH₂ (Compound 11; SEQ ID NO:11), optionally wherein the peptide has a disulfide bond formed between the thiol groups of two cysteine residues;
(g) Isovaleric acid-DTHFPCI(K(Palm))FGPRSKGWVCK-NH₂ (Compound 14; SEQ ID NO:14), optionally wherein the peptide has a disulfide bond formed between the thiol groups of two cysteine residues;
(h) Isovaleric acid-DTHFPCIKF(K(Palm))PRSKGWVCK-NH₂ (Compound 15; SEQ ID NO:15), optionally wherein the peptide has a disulfide bond formed between the thiol groups of two cysteine residues;
(i) Isovaleric acid-DTHFPCIKFGP(K(Palm))SKGWVCK-NH₂ (Compound 16; SEQ ID NO:16), optionally wherein the peptide has a disulfide bond formed between the thiol groups of two cysteine residues;
(j) Isovaleric acid-DTHFPCIKFGPRSKGWVC(K(Palm))-NH₂ (Compound 18; SEQ ID NO:18), optionally wherein the peptide has a disulfide bond formed between the thiol groups of two cysteine residues;
(k) Isovaleric acid-DTHFPCI(K(PEG3-Palm))FGPRSKGWVCK-NH₂ (Compound 19; SEQ ID NO:19), optionally wherein the peptide has a disulfide bond formed between the thiol groups of two cysteine residues;
(l) Isovaleric acid-DTHFPCIKF(K(PEG3-Palm))PRSKGWVCK-NH₂ (Compound 20; SEQ ID NO:20), optionally wherein the peptide has a disulfide bond formed between the thiol groups of two cysteine residues;
(m) Isovaleric acid-DTHFPCIKFGP(K(PEG3-Palm))SKGWVCK-NH₂ (Compound 21; SEQ ID NO:21), optionally wherein the peptide has a disulfide bond formed between the thiol groups of two cysteine residues;
(n) Isovaleric acid-DTHFPCIKFGPRS(K(PEG3-Palm))GWVCK-NH₂ (Compound 22; SEQ ID NO:22), optionally wherein the peptide has a disulfide bond formed between the thiol groups of two cysteine residues;
(o) Isovaleric acid-DTHFPCIKFGPRSKGWVC(K(PEG3-Palm))-NH₂ (Compound 23; SEQ ID NO:23), optionally wherein the peptide has a disulfide bond formed between the thiol groups of two cysteine residues;
(p) Isovaleric acid-DTHFPCIKFGPRSKGWVC(K(PEG8))-NH₂ (Compound 24; SEQ ID NO:24), optionally wherein the peptide has a disulfide bond formed between the thiol groups of two cysteine residues;
(q) Isovaleric acid-DTHFPCIKF(K(isoGlu-Palm))PRSKGCK-NH₂ (Compound 26; SEQ ID NO:26), optionally wherein the peptide has a disulfide bond formed between the thiol groups of two cysteine residues;
(r) Isovaleric acid-DTHFPCIKFEP(K(isoGlu-Palm))SKGCK-NH₂ (Compound 27; SEQ ID NO:27), optionally wherein the peptide has a disulfide bond formed between the thiol groups of two cysteine residues;
(s) Isovaleric acid-DTHFPCIKFEPRS(K(isoGlu-Palm))GCK-NH₂ (Compound 28; SEQ ID NO:28), optionally wherein the peptide has a disulfide bond formed between the thiol groups of two cysteine residues;
(t) Isovaleric acid-DTHFPCI(K(Dapa-Palm))FEPRSKGCK-NH₂ (Compound 32; SEQ ID NO:32), optionally wherein the peptide has a disulfide bond formed between the thiol groups of two cysteine residues; or
(u) Isovaleric acid-DTHFPCIKFEP(K(Dapa-Palm))SKGCK-NH₂ (Compound 34; SEQ ID NO:34), optionally wherein the peptide has a disulfide bond formed between the thiol groups of two cysteine residues.

9. The hepcidin mimetic for use of claim 4, wherein the peptide is selected from the group consisting of:
(a) 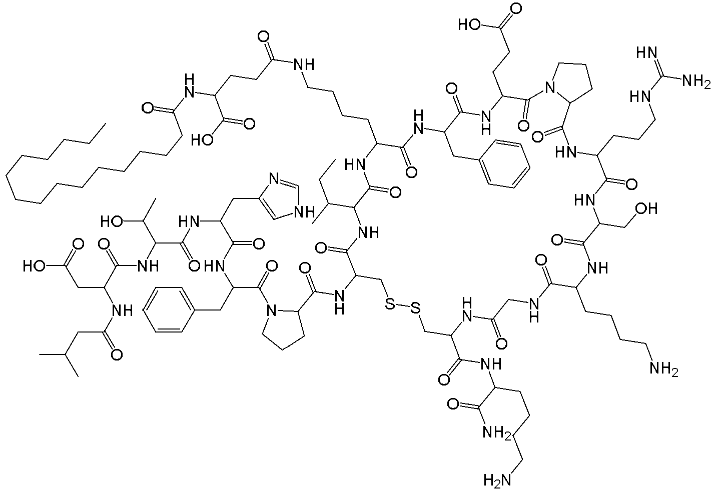 Isovaleric acid-DTHFPCI(K(isoGlu-Palm))FEPRSKGCK-NH₂ (Compound 25; SEQ ID NO:25);
(b) 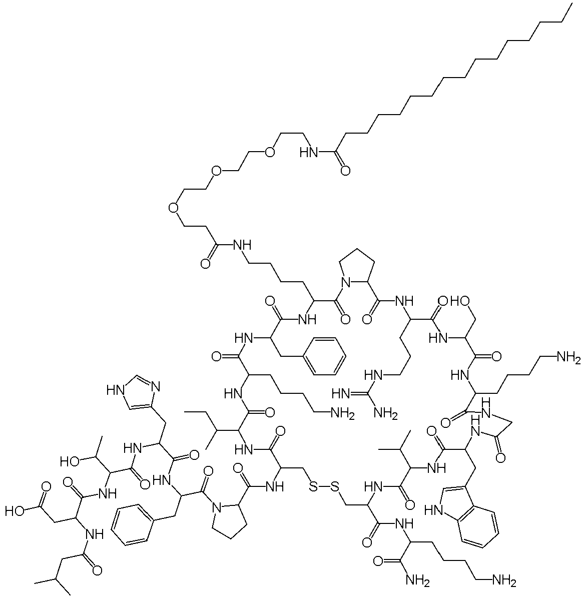 Isovaleric acid-DTHFPCIKF(K(PEG3-Palm))PRSKGWVCK-NH₂ (Compound 20; SEQ ID NO:20);
(c) 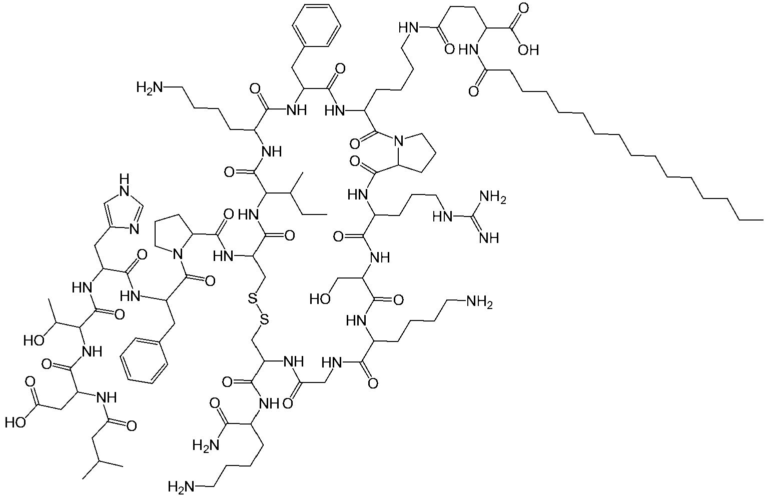 Isovaleric acid-DTHFPCIKF(K(isoGlu-Palm))PRSKGCK-NH₂ (Compound 26; SEQ ID NO:26);
(d) 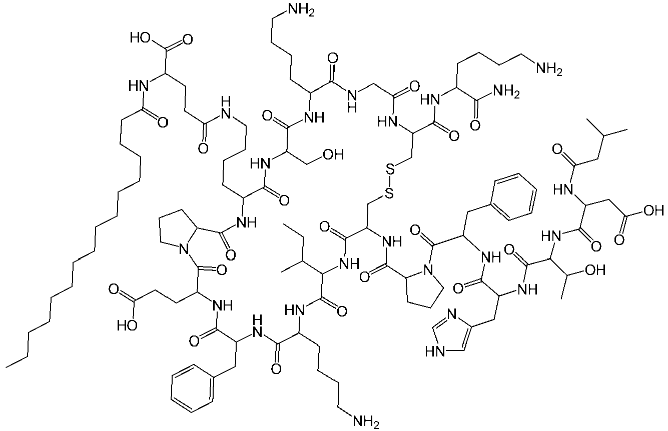 Isovaleric acid-DTHFPCIKFEP(K(isoGlu-Palm))SKGCK-NH₂ (Compound 27; SEQ ID NO:27); and
(e) 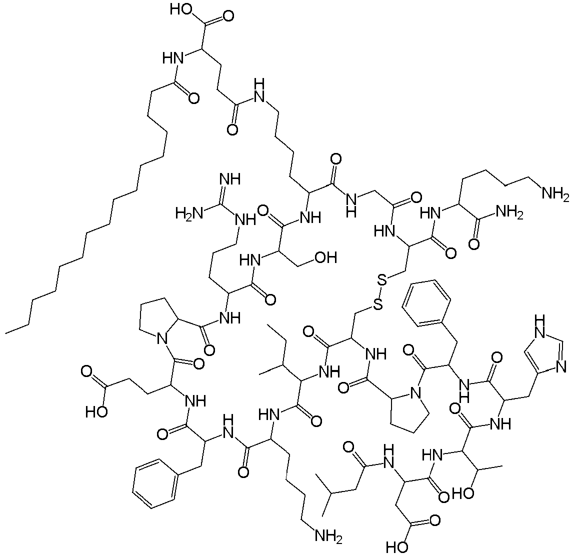 Isovaleric acid-DTHFPCIKFEPRS(K(isoGlu-Palm))GCK-NH₂ (Compound 28; SEQ ID NO:28),
wherein the amino acids are L-amino acids.

10. The hepcidin mimetic for use of any one of claims 1-9, wherein the method comprises measuring transferrin saturation (TSAT) and/or serum iron level in the subject before and after the hepcidin mimetic is administered to the subject, optionally wherein the TSAT level is measured at trough level of the hepcidin mimetic following administration to the subject;
optionally wherein the subject's TSAT level and/or serum iron level is decreased, further optionally wherein the subject's TSAT level is decreased to less than 45% or to less than 40%; still further optionally wherein the subject's TSAT level is maintained at less than 45% over the course of treatment with the hepcidin mimetic, optionally wherein the course of treatment comprises at least 24 weeks.

11. The hepcidin mimetic for use of any one of claims 1-10, wherein the hepcidin mimetic is administered to the subject subcutaneously.

12. The hepcidin mimetic for use of any one of claims 1-11, wherein the subject received phlebotomies for at least six months prior to treatment, optionally with a phlebotomy frequency of 0.25 - 1 phlebotomy per month;
further optionally wherein during the treatment, the subject required substantially fewer or no phlebotomies, optionally with a phlebotomy frequency of less than 0.1, less than 0.05, or no phlebotomies per month.

13. A compound having the formula: 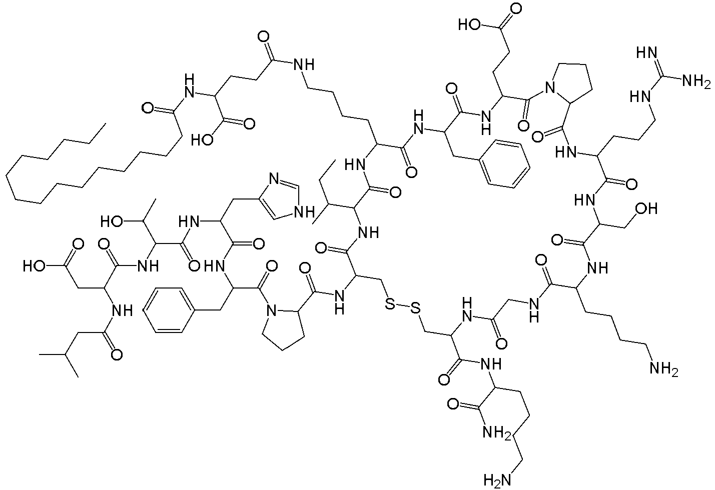 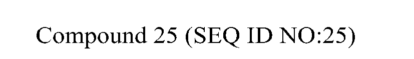 or a pharmaceutically acceptable salt thereof, or a peptide having the sequence: Isovaleric acid-DTHFPCI(K(isoGlu-Palm))FEPRSKGCK-NH₂ (SEQ ID NO:25), or a pharmaceutically acceptable salt thereof, wherein the thiol groups of two cysteine residues in the peptide optionally form a disulfide bond; for use in a method of treating hereditary hemochromatosis in a human subject, the method comprising administering to the subject an effective amount of the compound, peptide, or pharmaceutically acceptable salt thereof, wherein the effective amount comprises a dose in the range of 5 mg to 40 mg, and optionally wherein the subject is administered different doses during different time periods over a course of treatment.

14. A hepcidin mimetic for use in a method of treating hereditary hemochromatosis arthropathy or joint pain associated with hereditary hemochromatosis arthropathy in a human subject, the method comprising administering to the subject an effective amount of the hepcidin mimetic;
optionally wherein the effective amount comprises a dose in the range of 5 mg to 40 mg, and further optionally wherein the subject is administered different doses during different time periods over a course of treatment.

15. The hepcidin mimetic for use of claim 14, wherein the hepcidin mimetic is Compound 25:
